# EUROPEAN PATENT APPLICATION

(11) **EP 4 524 244 A1**
(43) Date of publication of application: **19.03.2025**
(21) Application number: 23803902.8
(22) Date of filing: 15.05.2023
(51) Int. Cl.: C12N 9/22, C12N 15/113

(54) **SEQUENCE DETERMINANTS OF DSRNA PROCESSING BY DICER**

(30) Priority: 13.05.2022 KR 20220059227
(71) Applicant: Seoul National University R&DB Foundation, Seoul 08826 (KR); Institute for Basic Science, Yuseong-gu Daejeon 34126 (KR)
(72) Inventor: KIM, V. Narry, Seoul 06276 (KR); LEE, Young-Yoon, Seoul 08787 (KR); KIM, Haedong, Seattle, Washington 98105 (US)
(74) Representative: J A Kemp LLP
(86) International application number: PCT/KR2023/006571
(87) International publication number: WO 2023/219479

(57) **Abstract**

The present invention relates to a DICER cleavage site motif that is a sequence determinant of dsRNA processing by DICER. Using the DICER cleavage site motif according to the present invention can strongly promote the processing of dsRNA by DICER, and thereby promote RNA interference. In addition, the DICER cleavage site motif according to the present invention is an integrated and conserved determinant of substrate recognition by DICER, and can be applied to any technique capable of generating siRNA through DICER processing. Thus, the present invention can greatly contribute to future studies using DICER processing, for example, studies on the biological or therapeutic use of small RNAs.

## Description

### Technical Field

The present disclosure was made under the support of the Ministry of Science and ICT of the Republic of Korea, under project number IBS-R008-D1-2022-A00. The research management agency for the project is the Institute for Basic Science (IBS), and the project title is "Support for Research Operation Costs of IBS." The research task is titled "Study on Cell Fate Regulation by RNA," with IBS as the principal institution. The research period was from January 1, 2022, to December 31, 2022.

This patent application claims the benefit of and priority to Korean Patent Application No. 10-2022-0059227, filed on May 13, 2022, with the Korean Intellectual Property Office, the disclosure of which is incorporated herein by reference.

The present disclosure relates to a DICER cleavage site motif, which is a sequence determinant of dsRNA processing by DICER.

### Background Art

DICER is one of the multidomain ribonuclease (RNase) III enzymes, which cleaves double-stranded RNA (dsRNA) into small RNAs of 21 to 25 nucleotides (nt) in length, playing a central role in RNA silencing. Endogenous siRNAs and miRNAs are generated from long RNA duplexes and hairpin RNAs, respectively. DICER processes pre-miRNAs to produce duplexes of approximately 22 nt with 2 nt 3' overhangs at both ends. After being loaded onto Argonaute (Ago) proteins, one strand of the duplex remains as the mature miRNA, serving as a guide that base-pairs with its cognate target. Even small changes in the processing site by DICER can alter the 'seed' sequence (the 2-7 nt region relative to the 5' end of the guide RNA), which is critical for target binding, meaning that the specificity of miRNAs to their targets is directly linked to the precision of pre-miRNA processing.

Currently, DICER is known to rely on secondary structural features of its dsRNA substrate, such as a 2 nt 3' overhang, a dsRNA stem of approximately 22 base pairs (bp), and a terminal loop, to recognize its substrates. According to this prevailing model, DICER functions as a 'molecular ruler' that measures 22 nt from the ends of pre-miRNAs. The 3' end is recognized by the conserved '3' pocket' in the PAZ domain within DICER, and some DICERs also have a '5' pocket' in the platform domain to capture the 5' end of the pre-miRNA. Because the catalytic center of DICER is located at a fixed distance from these pockets, DICER can measure the specified length (22 nt in the case of human DICER) from the 5' and 3' ends (referred to as the 5' counting rule and 3' counting rule). However, these 5' and 3' counting rules cannot fully explain DICER's processing patterns. Previous studies on DICER's substrate specificity have focused on the secondary structure of dsRNA, but there is a lack of research on other important factors influencing DICER's substrate specificity.

### Disclosure of Invention

### Technical Problem

Leading to the present disclosure, intensive and thorough research conducted by the present inventors with the aim of identifying the substrate specificity determinants of dsRNA processing by DICER resulted in the finding that DICER recognizes specific motifs with particular sequences within dsRNA and processes the dsRNA accordingly.

Thus, the present disclosure aims primarily to provide a DICER cleavage site motif.

Also, the present disclosure is to provide a method for promoting RNA interference.

Furthermore, the present disclosure is to provide a method for promoting homogeneous processing of dsRNA.

### Solution to Problem

Provided according to one aspect of the present disclosure is a DICER cleavage site motif including a 5' arm with a nucleic acid sequence of 5'-N₁N₂N₃-3' and a 3' arm with a nucleic acid sequence of 5'-N_{3'}N_{2'}N_{1'}-3', wherein the cleavage by DICER occurs between N3' and N2' of the 3' arm and the pair of nucleic acid sequences, 5'-N₁N₂N₃-3' and 5'-N_{3'}N_{2'}N_{1'}-3', that constitute the DICER cleavage site motif is selected from the group consisting of the following nucleic acid sequence pairs:
5'-CGC-3'/5'-GCG-3';
5'-AGC-3'/5'-GCA-3';
5'-CGC-3'/5'-GCC-3';
5'-CAC-3'/5'-GUC-3';
5'-AAU-3'/5'-AUU-3';
5'-CGC-3'/5'-GUC-3';
5'-AGC-3'/5'-GCU-3';
5'-AGU-3'/5'-ACA-3';
5'-AAC-3'/5'-GUA-3';
5'-AGC-3'/5'-GCG-3';
5'-GGC-3'/5'-GCG-3';
5'-AAC-3'/5'-GUC-3';
5'-UAC-3'/5'-GCA-3';
5'-UUC-3'/5'-GUA-3';
5'-AUU-3'/5'-AAA-3';
5'-UGC-3'/5'-GCC-3';
5'-AGC-3'/5'-GCC-3';
5'-CGG-3'/5'-CUC-3';
5'-UGC-3'/5'-GCA-3';
5'-CAC-3'/5'-GUG-3';
5'-CGU-3'/5'-ACC-3';
5'-AAC-3'/5'-GUU-3';
5'-GGC-3'/5'-GCA-3';
5'-AAC-3'/5'-GCU-3';
5'-CAU-3'/5'-AUC-3';
5'-AUC-3'/5'-GAU-3';
5'-UGC-3'/5'-GCU-3';
5'-UGC-3'/5'-GUC-3';
5'-UUC-3'/5'-GUG-3';
5'-CGC-3'/5'-GCU-3';
5'-AGC-3'/5'-GUC-3';
5'-ACC-3'/5'-GGA-3';
5'-UUC-3'/5'-GAA-3';
5'-CUC-3'/5'-GAC-3';
5'-CUU-3'/5'-AAC-3';
5'-AAC-3'/5'-GUG-3';
5'-CCC-3'/5'-GGC-3';
5'-AGU-3'/5'-ACC-3';
5'-CAC-3'/5'-GCG-3';
5'-AGU-3'/5'-ACG-3'; and
5'-UAC-3'/5'-GUA-3'.

The present inventors conducted research to identify determinants of substrate specificity for dsRNA processing by DICER, beyond the secondary structure of dsRNA, which culminated in discovering that DICER recognizes specific motifs within dsRNA, termed the 'DICER cleavage site motif,' and processes the dsRNA accordingly.

Specifically, as demonstrated in the examples described later, the inventors randomized the sequence of a specific region in the upper stem of pre-miRNAs and performed large-scale parallel analysis to identify position-dependent sequence determinants of pre-miRNA processing by DICER. As a result, a position-dependent 3-bp motif was identified to strongly promote DICER processing, termed the 'DICER cleavage site motif,' which exists in the region from the -1 to +1 position relative to the cleavage site of the 3p strand. The region from the -1 to +1 position relative to the cleavage site of the 3p strand corresponds to the 5'-N_{3'}N_{2'}N_{1'}-3' of the 3' arm in the aforementioned DICER cleavage site motif. In the context of this disclosure, unless specifically stated otherwise, the numbering used to indicate the position of the DICER cleavage site is based on the cleavage site of the 3' arm. During the process of identifying the 'DICER cleavage site motif,' the processing efficiency of DICER for each 3-bp motif was quantified using a metric termed the 'cleavage score.' The 'cleavage score' is calculated by dividing the proportion of each 3-bp motif in the input variant population (Fraction of input*_{variant i}*) by the proportion in the uncleaved pre-miRNA sample after the reaction (Fraction of uncleaved*_{variant i}*).

Among the position-dependent 3-bp motifs that strongly promote DICER processing, many tended to exhibit the following regularity: a paired guanine (G) at the -1 position relative to the cleavage site of the 3p strand; a paired pyrimidine (Y) (cytosine (C) or uridine (U)) at the 0 position relative to the cleavage site of the 3p strand; and a mismatch (M) at the +1 position relative to the cleavage site of the 3p strand. Based on this tendency, the inventors conveniently named the 3-bp motif as the "GYM motif," which is used interchangeably with "DICER cleavage site motif." It should be noted that a GYM motif that strongly promotes DICER processing does not necessarily include the aforementioned "GYM" sequence (G, Y, and M at the -1 to 0 positions of the 3p strand) and may include other nucleic acid sequences, as will be clearly understood from the results of the Example section described later. For instance, in the case of the motif 5'-CGC-3' (5' arm)/5'-GCG-3' (3' arm), which has the highest "GYM score" as a measure of DICER cleavage, it does not contain a mismatch (M). Additionally, it should be noted that the "GYM motif" should be recognized as a quantitative characteristic based on cleavage scores rather than being strictly defined according to the aforementioned consensus sequence. Therefore, the inventors devised the "GYM score" as an indicator that can reflect this quantitative characteristic of the motif. Herein, the GYM score refers to a value obtained by (1) identifying the reaction time at which 20% of the substrates pre-let-7a-1 and pre-miR-374b are cleaved, (2) measuring the cleavage score of each 3-bp motif, i.e., DICER cleavage site motif, at the identified reaction time, (3) calculating the average of the cleavage scores for the two substrates (pre-let-7a-1 and pre-miR-374b) with each 3-bp motif, and (4) normalizing the average value to a score ranging from 0 to 100.

The nucleic acid sequence pairs listed above represent in the 5' arm/3' arm order the top 1% of nucleic acid sequence pairs with the highest GYM scores among the 3-bp motifs at the -1 to +1 positions on the 3p side of all pre-miRNAs investigated in the following examples. The cleavage scores and GYM scores for each nucleic acid sequence pair are shown in Table 1 below.

**TABLE**

| Nucleic acid sequence pair 5'-N₁N₂N₃-3' (5' arm) / 5' -N_{3'}N_{2'}N_{1'}-3' (3' arm) | Cleavage Score at 20% cleavage for pre-let-7a-1 | Cleavage Score at 20% cleavage for pre-miR-374b | Average Cleavage Score | GYM Score |
|---|---|---|---|---|
| CGC/GCG | 15.1998091 | 16.9373336 | 16.0685713 | 100 |
| AGC/GCA | 13.9313191 | 17.608406 | 15.7698626 | 98.1410373 |
| CGC/GCC | 16.1872842 | 13.9615031 | 15.0743936 | 93.8129056 |
| CAC/GUC | 17.0408108 | 12.1525437 | 14.5966772 | 90.8399194 |
| AAU/AUU | 12.0662878 | 15.4181213 | 13.7422046 | 85.5222551 |
| CGC/GUC | 16.3613152 | 7.82556757 | 12.0934414 | 75.26146 |
| AGC/GCU | 10.1253314 | 13.2063491 | 11.6658403 | 72.6003578 |
| AGU/ACA | 9.57612262 | 13.7358989 | 11.6560108 | 72.5391854 |
| AAC/GUA | 11.8319464 | 11.379617 | 11.6057817 | 72.2265934 |
| AGC/GCG | 8.34949951 | 14.7011323 | 11.5253159 | 71.7258285 |
| GGC/GCG | 10.3083987 | 12.7401807 | 11.5242897 | 71.7194418 |
| AAC/GUC | 9.48648451 | 13.4472285 | 11.4668565 | 71.3620164 |
| UAC/GCA | 10.0504991 | 12.7626387 | 11.4065689 | 70.9868266 |
| UUC/GUA | 11.5896467 | 10.9282107 | 11.2589287 | 70.0680133 |
| AUU/AAA | 13.380438 | 8.86192659 | 11.1211823 | 69.2107722 |
| UGC/GCC | 11.0788085 | 10.3579091 | 10.7183588 | 66.7038692 |
| AGC/GCC | 8.94237193 | 12.4681206 | 10.7052463 | 66.6222656 |
| CGG/CUC | 9.18508731 | 12.0615227 | 10.623305 | 66.1123181 |
| UGC/GCA | 12.1721025 | 8.80341191 | 10.4877572 | 65.2687598 |
| CAC/GUG | 10.4615394 | 10.3055325 | 10.383536 | 64.6201566 |
| CGU/ACC | 10.2913318 | 10.2200181 | 10.2556749 | 63.8244355 |
| AAC/GUU | 11.9894869 | 8.20029354 | 10.0948902 | 62.8238192 |
| GGC/GCA | 6.96164533 | 13.1373993 | 10.0495223 | 62.54148 |
| AAC/GCU | 7.54188799 | 12.4901849 | 10.0160364 | 62.3330864 |
| CAU/AUC | 10.5006257 | 9.42230654 | 9.96146612 | 61.9934773 |
| AUC/GAU | 13.0009508 | 6.78553583 | 9.89324331 | 61.5689043 |
| UGC/GCU | 8.1802529 | 11.4326553 | 9.80645411 | 61.0287866 |
| UGC/GUC | 9.34712439 | 10.2309172 | 9.78902077 | 60.9202932 |
| UUC/GUG | 9.42471328 | 10.0570245 | 9.74086889 | 60.6206282 |
| CGC/GCU | 9.13334362 | 10.052614 | 9.59297881 | 59.7002597 |
| AGC/GUC | 6.76986447 | 12.3196098 | 9.54473715 | 59.400036 |
| ACC/GGA | 7.71623998 | 11.3339653 | 9.52510265 | 59.277844 |
| UUC/GAA | 13.1746872 | 5.47992603 | 9.32730663 | 58.0468944 |
| CUC/GAC | 8.82737229 | 9.82684298 | 9.32710764 | 58.045656 |
| CUU/AAC | 8.35106118 | 10.2386018 | 9.29483147 | 57.8447908 |
| AAC/GUG | 6.53150943 | 11.3007561 | 8.91613279 | 55.4880244 |
| CCC/GGC | 9.84489799 | 7.55286205 | 8.69888002 | 54.1359891 |
| AGU/ACC | 7.22301969 | 10.1737272 | 8.69837343 | 54.1328364 |
| CAC/GCG | 5.97340982 | 11.3748603 | 8.67413505 | 53.981993 |
| AGU/ACG | 5.09539055 | 12.2429318 | 8.66916116 | 53.9510388 |
| UAC/GUA | 12.5115784 | 4.77107988 | 8.64132914 | 53.777831 |

As used herein, the term "nucleic acid" refers to a polymer of deoxyribonucleotides, ribonucleotides, or modified nucleotides, and can be in single-stranded or double-stranded form. The term is intended to encompass nucleic acids containing known nucleotide analogs or modified backbone residues or linkages, whether synthetic, naturally occurring, or non-naturally occurring, which have binding properties similar to standard nucleic acids and are metabolized in a similar manner to standard nucleotides. Examples of such analogs include, but are not limited to, phosphorothioate, phosphoramidate, methyl phosphonate, chiral-methyl phosphonate, 2'-O-methyl ribonucleotides, and peptide nucleic acids (PNA).

As used herein, the term "nucleotide" is intended to encompass those with natural bases (standard) and modified bases that are well known in the art. These bases typically reside at the 1' position of the nucleotide sugar moiety. Nucleotides generally comprise a base, sugar, and phosphate group. The nucleotide may be unmodified or modified in the sugar, phosphate, and/or base moiety, or omitted, and may also be referred to interchangeably as a nucleotide analog, modified nucleotide, non-natural nucleotide, or non-standard nucleotide [see references: for example, Usman and McSwiggen, supra; Eckstein, et al., International PCT Publication No. WO 92/07065; Usman et al, International PCT Publication No. WO 93/15187; Uhlman & Peyman, supra, all of which are incorporated herein by reference]. Various examples of modified nucleotide bases known in the art are summarized in the literature [see reference: Limbach, et al, Nucleic Acids Res. 22:2183, 1994]. Nonlimiting examples of base modifications that can be introduced into nucleic acid molecules include hypoxanthine, purine, pyridin-4-one, pyridin-2-one, phenyl, pseudouracil, 2,4,6-trimethoxy benzene, 3-methyl uracil, dihydrouridine, naphthyl, aminophenyl, 5-alkylcytidine (e.g., 5-methylcytidine), 5-alkyluridine (e.g., ribothymidine), 5-halogeno-uridine (e.g., 5-bromouridine), or 6-azapyrimidine or 6-alkylpyrimidine (e.g., 6-methyluridine), and propyne, among others (see reference: Burgin, et al., Biochemistry 35:14090, 1996; Uhlman & Peyman, supra). In this context, the term "modified base" refers to nucleotide bases other than adenine, guanine, cytosine, and uracil at the 1' position or equivalent positions.

The term "double-stranded RNA (dsRNA)", as used herein, refers to a molecule comprising two oligonucleotide strands that form a duplex. The dsRNA in this disclosure may include ribonucleotides as well as ribonucleotides, deoxyribonucleotides, modified nucleotides, or combinations thereof. The double-stranded RNA of the present disclosure can serve as a substrate for proteins or protein complexes in the RNA interference pathway, such as DICER or RISC (RNA-induced silencing complex).

As used herein, the term "duplex" refers to a double-helical structure formed by the interaction of two single-stranded nucleic acids. A duplex is typically formed by base-pairing hydrogen bonds, i.e., "base pairing," between two single-stranded nucleic acids that are oriented antiparallel to each other. Base-pairing in a duplex generally occurs through Watson-Crick or wobble base pairing, e.g., guanine (G) pairs with cytosine (C) in both DNA and RNA (thus, the cognate nucleotide of guanine deoxyribonucleotide is cytosine deoxyribonucleotide, and vice versa), and adenine (A) pairs with thymine (T) in DNA and with uracil (U) in RNA. Another example of wobble base pairing is guanine (G) pairing with uracil (U). The conditions under which these base pairs can form include physiological or biologically relevant conditions (e.g., intracellular: pH 7.2, 140mM potassium ions; extracellular: pH 7.4, 145mM sodium ions). Additionally, the duplex is stabilized by stacking interactions between adjacent nucleotides. As used herein, a duplex can be established and maintained by base pairing or stacking interactions. A duplex may be formed by two complementary nucleic acid strands that are either substantially or fully complementary.

The term "Complementary" or "complementarity", as used herein, refers to the ability of a nucleic acid to form hydrogen bonds with another nucleic acid sequence through conventional Watson-Crick, wobble, or Hoogsteen base pairing. Regarding the nucleic acid molecules of the present disclosure, the binding free energy of the nucleic acid molecule and its complementary sequence is sufficient to exhibit the relevant function of the nucleic acid, such as RNAi activity. The measurement of binding free energy of nucleic acid molecules is well known in the art [see reference: for example, Turner, et al., CSH Symp. Quant. Biol. LII, pp. 123-133, 1987; Frier, et al., Proc. Nat. Acad. Sci. USA 83:9373-9377, 1986; Turner, et al., J. Am. Chem. Soc. 109:3783-3785, 1987]. The % complementarity refers to the % of consecutive residues within a nucleic acid molecule that can form hydrogen bonds (e.g., Watson-Crick base pairing) with a second nucleic acid sequence (e.g., 50%, 60%, 70%, 80%, 90%, or 100% complementarity if 5, 6, 7, 8, 9, or 10 nucleotides out of 10 in a first oligonucleotide form base pairs with a nucleic acid sequence of 10 nucleotides, respectively). To determine whether the % complementarity exceeds a specific %, the % of consecutive residues within a nucleic acid molecule that can form hydrogen bonds (e.g., Watson-Crick base pairing) with a second nucleic acid sequence is approximately calculated to the nearest integer (e.g., 52%, 57%, 61%, 65%, 70%, 74% if 12, 13, 14, 15, 16, or 17 nucleotides out of 23 in a first oligonucleotide form base pairs with a second nucleic acid sequence of 23 nucleotides, corresponding to at least 50%, 50%, 60%, 60%, 70%, and 70% complementarity, respectively). As used herein, "substantially complementary" refers to complementarity between strands that allows them to hybridize under physiological conditions. Substantially complementary sequences have 60%, 70%, 80%, 90%, 95%, or even 100% complementarity. Additionally, techniques for determining whether two strands can hybridize under physiological conditions by examining nucleotide sequences are well known in the art. In the present disclosure, the first and second strands (antisense and sense oligonucleotides) need not be fully complementary and may contain mismatches.

The term "5' arm", as used herein, refers to the strand where the 3' end is formed at the DICER cleavage site, with the 5' end exposed on the opposite side.

The term "3' arm", as used herein, refers to the strand where the 5' end is formed at the DICER cleavage site, with the 3' end exposed on the opposite side.

Throughout this disclosure, complementary binding between specific nucleic acids of the 5' arm and specific nucleic acids of the 3' arm that form base pairs (double-stranded) may be represented as 5'-N₁N₂N₃-3'/5'-N_{3'}N_{2'}N_{1'}-3', which does not imply that the two nucleic acids constitute a single strand from the 5' end to the 3' end.

In the present disclosure, the 5' arm and 3' arm may be two separate strands, but they do not necessarily need to be; a single nucleic acid strand containing a loop structure may form the double-stranded stem portion.

As used herein, the term "DICER" refers to an RNase III family endonuclease that cleaves dsRNA or dsRNA-containing molecules, such as dsRNA or pre-miRNA, into double-stranded nucleic acid fragments approximately 19 to 25 nucleotides in length. DICER typically contains two RNase III domains that cleave both the sense and antisense strands of dsRNA. The average distance between the RNase III domain and the PAZ domain determines the length of the short double-stranded nucleic acid fragments generated, and this distance can vary (see: Macrae I, et al. (2006). "Structural basis for double-stranded RNA processing by Dicer". Science 311 (5758): 195-8.).

The term "DICER cleavage site", as used herein, refers to the site where DICER cleaves dsRNA. The DICER cleavage site is characterized by the cleavage occurring such that a 2 nt overhang is exposed at the 3' end of the 5' arm. Specifically, the cleavage occurs between N3' and N2' of the sequence 5'-N_{3'}N_{2'}N_{1'}-3' based on the 3' arm, while from the perspective of the 5' arm, the cleavage occurs 2 nt downstream from N2 towards the 3' end of the sequence 5'-N₁N₂N₃-3'.

The term "motif", as used herein, refers to a recurring pattern or sequence of nucleotides, amino acids, or other molecular features that are conserved in a molecule or organism.

The term "DICER cleavage site motif", as used herein, refers to a specific motif recognized by DICER when cleaving dsRNA, as described above, and means the region from the -1 to +1 position relative to the cleavage site of the 3p strand.

The term "mismatch", as used herein, refers to the pairing of bases that cannot form complementary hydrogen bonds, meaning they are not fully complementary. Specifically, in dsRNA, a mismatch refers to the presence of bases in the two RNA strands that are not perfectly complementary at one or more positions in the dsRNA formed thereby.

In one embodiment of the present disclosure, the DICER cleavage site motif is recognized by the dsRBD (double-stranded RNA binding domain) of DICER. As demonstrated in Example 3 described later, DICER recognizes the DICER cleavage site motif through the dsRBD within DICER, thereby efficiently and precisely cleaving dsRNA.

The term "dsRBD" (double-stranded RNA binding domain), as used herein, refers to a conserved structural motif found in many RNA-binding proteins, including DICER. The dsRBD within DICER plays a role in recognizing and binding to dsRNA substrates, stabilizing the interaction between the enzyme and the substrate, and interacting with the helical structure of dsRNA. The dsRBD within DICER is critical for recognizing and processing dsRNA substrates, which are essential for the biogenesis of small RNA molecules, and mutations or defects in the dsRBD can affect the function of DICER and the production of small RNA molecules.

The term "recognition", as used herein, refers to the ability of a protein or other biomolecule to selectively bind to a specific sequence or structural motif in another molecule.

In one embodiment of the present disclosure, the DICER cleavage site motif is included in double-stranded RNA (dsRNA) and promotes the processing of the dsRNA by DICER. More specifically, the aforementioned dsRNA may be selected from the group consisting of pri-miRNA (primary miRNA), pre-miRNA (precursor-miRNA), shRNA (short hairpin RNA), DsiRNA (Dicer-substrate short interfering RNA), and long dsRNA, but with no limitations thereto.

As used herein, the term "pri-miRNA" (primary miRNA) refers to the primary precursor generated during the biogenesis of microRNA (miRNA) in eukaryotic cells, which is primarily transcribed from DNA by RNA polymerase II and refers to a precursor having a stem-loop structure similar to a hairpin structure.

As used herein, "pre-miRNA" (precursor-miRNA) refers to an RNA molecule that acts as an intermediate in the biogenesis of miRNA, and includes a shorter hairpin-structured RNA molecule generated in the nucleus by the cleavage of pri-miRNA by DROSHA.

When the dsRNA containing the DICER cleavage site motif of the present disclosure is a pri-miRNA, the pri-miRNA must include the motif so that the DICER cleavage site motif is preserved during the formation of pre-miRNA (precursor-miRNA) by cleavage by Drosha. More specifically, for example, the DICER cleavage site motif may be included so that cleavage by DICER can occur between the nucleic acid at approximately the 22 nt position from the 5' end of the 5' arm or the 3' end of the 3' arm of the formed pre-miRNA and the nucleic acid at the next position bound thereto.

As used herein, the term "shRNA" (short hairpin RNA) refers to an artificial RNA molecule with a tight hairpin turn that can be used to silence target gene expression through RNA interference (RNAi). The expression of shRNA in cells is generally carried out through the delivery of plasmids or via viral or bacterial vectors. shRNA is a favorable medium for RNAi due to its relatively low degradation and turnover rates.

When the dsRNA containing the DICER cleavage site motif of the present disclosure is shRNA, it can be designed and provided with a structure similar to that of pre-miRNA. The 3' end of the shRNA molecule may include a varying number of uridines derived from the RNA polymerase III termination signal. The siRNA guide sequence that forms complementary binding with the target nucleic acid sequence may be included in either the 3' arm or the 5' arm, and if the guide sequence is included in the 3' arm, the siRNA formed will include the N2'N1' sequence of the 5'-N_{3'}N_{2'}N_{1'}-3' at the 5' end, and it should be noted that various numbers of uridines may be included at the 3' end, as described above, to design and manufacture the shRNA molecule. If the guide sequence is included in the 5' arm, the siRNA formed will include the sequence 5'-N₁N₂N₃-3' at the 3' end or a sequence further comprising an additional nucleic acid sequence N of 1 to 2 nt at the 3' end of the sequence 5'-N₁N₂N₃-3', so the shRNA molecule can be designed and manufactured accordingly.

As used herein, the term "DsiRNA" (Dicer-substrate short interfering RNA) refers to a double-stranded RNA (dsRNA) molecule with a nucleotide length of 25 bp to 35 bp, which is processed by DICER in the RNA interference (RNAi) pathway and means dsRNA used as an alternative to conventional 21-mer siRNA. Unlike the aforementioned pri-miRNA, pre-miRNA, and shRNA, DsiRNA consists of two separate strands forming complementary binding, but it can be processed by DICER in a similar manner, and thus, the DICER cleavage site motif of the present disclosure can be suitably applied.

Even when the dsRNA containing the DICER cleavage site motif of the present disclosure is DsiRNA, guide RNA sequences can be selectively included in the 5' arm and 3' arm, similar to the other dsRNAs described above, and the sequences can be designed and manufactured with reference to the detailed description provided for shRNA.

As used herein, the term "long dsRNA" refers to dsRNA longer than 22 bp, which plays a role in regulating gene expression in eukaryotic cells. The long dsRNA of the present disclosure may also be applied in a manner similar to the aforementioned DsiRNA.

The DICER cleavage site motif may be artificially introduced into dsRNA to regulate the efficiency, accuracy, and position of dsRNA processing by DICER.

In one embodiment of the present disclosure, the DICER cleavage site motif promotes the cleavage of the dsRNA by DICER, thereby promoting processing by DICER.

In one embodiment of the present disclosure, the DICER cleavage site motif promotes the biogenesis of miRNA or siRNA. The DICER cleavage site motif of the present disclosure pertains to the top 1% of DICER cleavage site motifs with a relatively elevated GYM score compared to typical wild-type DICER cleavage sites, and it promotes the generation of miRNA and siRNA through cleavage by DICER.

In one embodiment of the present disclosure, the DICER cleavage site motif promotes RNA interference. As described in the foregoing, the DICER cleavage site motif of the present disclosure not only promotes the generation of miRNA and siRNA but also significantly increases complementary binding with the target RNA molecule, thereby promoting RNA interference.

In an embodiment of the present disclosure, the DICER cleavage site motif position and GYM motif characteristics are commonly identified and applied in metazoans.

As used herein, the term "metazoa" refers to animals belonging to the kingdom Animalia, which includes all multicellular animals that form tissues through cell differentiation, excluding protozoa. For example, metazoans may refer to animals belonging to the phyla Cnidaria, Annelida, Arthropoda, Chordata, Nematoda, Platyhelminthes, or Mollusca, but with no limitations thereto.

According to another aspect thereof, the present disclosure provides a dsRNA nucleic acid molecule including the "DICER cleavage site motif" of another aspect of the present disclosure as described above.

As used herein, the term "dsRNA" used to describe the "dsRNA nucleic acid molecule" of the present disclosure has been described in detail above.

In one embodiment of the present disclosure, the dsRNA nucleic acid molecule of the present disclosure includes a stem of 22 bp or longer, and the DICER cleavage site motif is included at positions 18 to 20, 19 to 21, or 20 to 22 from the 5' end.

The stem forms a double strand with or without one or more mismatches, and the dsRNA nucleic acid molecule generates miRNA or siRNA of 21 nt to 23 nt in length by cleavage by DICER.

In another embodiment of the present disclosure, the dsRNA nucleic acid molecule of the present disclosure is selected from the group consisting of pri-miRNA (primary miRNAs), pre-miRNA (precursor-miRNA), shRNA (short hairpin RNA), DsiRNA (Dicer-substrate short interfering RNA), and long dsRNA.

The dsRNA of the present disclosure can be suitably used as an alternative to overcome the disadvantages of conventional siRNA, and with the DICER cleavage site motif introduced thereinto considering the GYM score, the dsRNA possesses the characteristic of promoting siRNA production and enhancing RNA-mediated interference.

The present disclosure relates to a dsRNA sequence comprising the "DICER cleavage site motif" of another aspect of the present disclosure as described above, and redundant content is hereby incorporated by reference. Redundant descriptions are omitted to avoid unnecessary complexity in the present disclosure.

Provided according to another aspect of the present disclosure is a method for enhancing the capacity of RNA interference (RNAi) of a target dsRNA, the method comprising the steps of:
(a) calculating the GYM score of the DICER cleavage site motif within the target dsRNA; and
(b) replacing the DICER cleavage site motif within the target dsRNA with a second DICER cleavage site motif having a relatively higher GYM score than the GYM score of the DICER cleavage site motif within the target dsRNA, calculated in step (a).

Below, a detailed description will be given in a stepwise manner of the present disclosure.

### (a) Step of calculating GYM score of DICER cleavage site motif within target dsRNA

The term "GYM score", as used herein, has been described in the foregoing and can be clearly understood through the examples provided later. To explain more specifically, the GYM score of the present disclosure is determined by (1) identifying the reaction time at which 20% of the DICER substrates pre-let-7a-1 and pre-miR-374b are cleaved, (2) measuring the cleavage score of each DICER cleavage site motif at the identified reaction time, (3) calculating the average of the cleavage scores for the two substrates (pre-let-7a-1 and pre-miR-374b) with each DICER cleavage site motif, and (4) normalizing the average value to a score ranging from 0 to 100. The cleavage score corresponds to the ratio of the fraction of each DICER cleavage site motif in the input variant population (Fraction of input*_{variant i}*) divided by the fraction in the uncleaved pre-miRNA sample after the reaction (Fraction of uncleaved*_{variant i}*).

### (b) Replacing DICER cleavage site motif within target dsRNA with a second DICER cleavage site motif having relatively higher GYM score than GYM score of the DICER cleavage site motif within the target dsRNA calculated in step (a)

The "target dsRNA" of the present disclosure may be a naturally occurring wild-type sequence nucleic acid molecule or a previously known artificial sequence nucleic acid molecule. The GYM motif at the predicted or identified DICER cleavage site of these nucleic acid molecules can be replaced with a second DICER nucleic acid sequence that has a relatively higher GYM score, thereby significantly enhancing the RNA interference capacity of the target dsRNA.

The sequences listed in Table 1 of the description are ordered from the highest to the lowest GYM score, and the sequences listed in Table 1 correspond to the top 1% of sequences with the highest GYM scores among all sequences. Nevertheless, the score difference between the sequence with the highest GYM score (5'-CGC-3' (5' arm)/5'-GCG-3' (3' arm)) and the sequence with the lowest GYM score listed in Table 1 (5'-UAC-3' (5' arm)/5'-GUA-3' (3' arm)) is approximately 46.2, showing a significant difference. Therefore, while it is possible to replace sequences in the remaining 99% lower range with those having relatively higher GYM scores within the same lower 99% range, a substantial increase in the GYM score can be achieved by replacing them with the sequences listed in Table 1 of this disclosure. Additionally, the capacity of RNA interference (RNAi) of the target dsRNA can be further enhanced by replacing even the sequences listed in Table 1 of this disclosure with other nucleic acid sequences having higher GYM scores.

Another aspect of the present disclosure provides a method for promoting homogeneous processing of double-stranded RNA (dsRNA), the method including the step of: (a) forming a DICER cleavage site motif at a cleavage site within the target dsRNA such that the desired nucleic acid molecule is generated by cleavage by DICER; wherein the DICER cleavage site motif is formed so that the GYM score thereof is relatively higher than the GYM scores of all nucleic acid sequences determined by 1 nt to 2 nt frame shifts in both directions relative to the DICER cleavage site motif region.

In an embodiment of the present disclosure, the DICER cleavage site motif is formed so that its GYM score is higher by a difference of 1 or more, 2 or more, 3 or more, 4 or more, 5 or more, 6 or more, 7 or more, 8 or more, 9 or more, or 10 or more compared to the GYM scores of all nucleic acid sequences determined by 1 nt to 2 nt frame shifts in both directions relative to the DICER cleavage site motif region, but with no limitations thereto.

As used herein, the term "homogeneous processing" refers to the situation where, if a specific DICER cleavage site motif within dsRNA has a higher GYM score compared to neighboring DICER cleavage site motifs (i.e., all 3 nt-long nucleic acid sequences determined by 1 nt to 2 nt frame shifts in both directions relative to the DICER cleavage site motif region), processing by DICER predominantly occurs at that DICER cleavage site motif, resulting in the generation of homogeneous small RNAs. The difference in GYM scores between the specific DICER cleavage site motif and the neighboring DICER cleavage site motifs can be 1 or more, 2 or more, 3 or more, 4 or more, 5 or more, 7 or more, 8 or more, 9 or more, or 10 or more, but is not limited thereto.

As demonstrated in Example 5 later, when the difference in GYM scores between multiple DICER cleavage site motifs within the target dsRNA is 8 or less (i.e. GYM score is similar), no DICER cleavage site motif is so 'prominent,' and thus homogeneous processing does not occur, leading to "alternative processing," where multiple types of small RNAs are generated from a single dsRNA. In this case, if one or more neighboring DICER cleavage sites are replaced with DICER cleavage sites having lower GYM scores, the desired DICER cleavage site becomes relatively 'prominent,' resulting in predominant processing by DICER at that site, thereby achieving homogeneous processing. Alternatively, if the desired specific DICER cleavage site motif is formed so that it has a relatively higher GYM score compared to neighboring DICER cleavage site motifs, the motif becomes dominant, leading to homogeneous processing.

The 'not-so-prominent' GYM motif compared to neighboring positions allows for alternative processing, generating multiple miRNA isoforms (isomiRs) and diversifying miRNA function, suggesting that such 'not-so-prominent' GYM motifs have been retained during metazoan evolution.

In an embodiment of the present disclosure, the formation of the DICER cleavage site in step (b) above includes not only the replacement of the nucleic acid sequence within the existing DICER cleavage site of the target dsRNA but also the replacement of nucleic acid sequences at neighboring positions.

The method for promoting homogeneous processing of double-stranded RNA (dsRNA) of the present disclosure is carried out using the DICER cleavage site motif, cleavage score, and GYM score explained above, and therefore, common content between the two is omitted to avoid unnecessary complexity in the present disclosure.

According to another aspect of the present disclosure provides a method for promoting alternative processing of double-stranded RNA (dsRNA), the method comprising the step of: (a) replacing nucleic acids in the DICER cleavage site motif region of the target dsRNA so that a difference in GYM score between the major DICER cleavage site motif with the highest GYM score and at least one adjacent DICER cleavage site motif (all 3 nt-long nucleic acid sequences determined by 1 nt to 2 nt frame shifts in both directions relative to the major DICER cleavage site motif region) is less than 8.

The method of the present disclosure utilizes the principle that in the absence of a distinctly prominent GYM score in the DICER cleavage site motif region, a small GYM score difference with neighboring DICER cleavage site motifs results in the generation of various alternatively processed nucleic acid sequences, rather than a single predominant siRNA or miRNA sequence.

In one embodiment of the present disclosure, the nucleic acids in the DICER cleavage site motif region can be replaced so that the GYM score difference between the DICER cleavage site motifs is less than 8, less than 7, less than 6, less than 5, less than 4, less than 3, less than 2, or less than 1, but with no limitations thereto.

The method for promoting alternative processing of double-stranded RNA (dsRNA) of the present disclosure is carried out using the DICER cleavage site motif, cleavage score, and GYM score described in the foregoing and therefore, common content between the two is omitted to avoid unnecessary complexity in the present disclosure.

### Advantageous Effects of Invention

The present disclosure provides a DICER cleavage site motif, a method for promoting RNA interference, and a method for promoting homogeneous processing of dsRNA.

Unlike previous studies on the substrate specificity of DICER, the present disclosure has, for the first time, identified that DICER processes dsRNA by recognizing a specific motif with a particular sequence within dsRNA, referred to as the 'DICER cleavage site motif,' in addition to the secondary structure of the dsRNA substrate.

When utilizing the DICER cleavage site motif of the present disclosure, the processing of dsRNA by DICER can be strongly promoted, thereby enhancing RNA interference.

Furthermore, the DICER cleavage site motif of the present disclosure serves as an integral and conserved determinant of substrate recognition by DICER, finding applications in all technologies that involve the generation of siRNA through DICER-mediated processing. As a result, the present disclosure can significantly contribute to future research involving DICER-mediated processing, such as studies on the biological or therapeutic applications of small RNAs.

### Brief Description of Drawings

FIG. 1a illustrates the mechanism of cleavage site choice by DICER.
FIG. 1b shows examples of cleavage site decision of pre-let-7a-1 and pre-miR-324.
FIG. 1c illustrates the impact of mismatch in cleavage site decision in vitro. "No-bulge pre-miR-324" was used for the assay to avoid the influence of the "bulge". A mismatch near the cleavage was replaced with a base-pair marked in shade. Cleavage sites and their corresponding products are marked with arrowheads. The 5' phosphate was radiolabeled as indicated by *.
FIG. 2a illustrates a schematic outline of the massively parallel assay.
FIG. 2b illustrates a substrate design for the first screening (n=1,048,576 variants), wherein sequence variation is introduced to five base pairs at the positions -1 to 3 relative to the starting position of 3p miRNA.
FIG. 2c shows massively parallel assay results from the first screening, in terms of structural (left panel) and sequence (middle and right panels) features of pre-miRNA variants with top 0.1% cleavage scores.
FIG. 2d shows a substrate design for the second screening (n=4,096 variants), specifically with sequence variation introduced to three base pairs at the positions -1 to +1.
FIG. 2e shows the distribution of the cleavage scores of variants measured from the second screening, with top five motifs in each backbone given. Analyses were carried out using data from the condition for which about 20% of substrates were cleaved, which is true also for FIGS. 2f to 2h. (rₛ: Spearman correlation coefficient) .
FIG. 2f illustrates enrichment of sequences in the pre-miRNA variants with top 1% cleavage scores. Expected proportions were calculated by assuming that the four bases have equal contributions to the total cleavage score of the top 1% the pre-miRNA variants.
FIG. 2g shows plots of structural impact on cleavage scores. G-U base pair was considered as a mismatch only when it is between mismatches. (p, pair; m, mismatch).
FIG. 2h shows diagrams of impact of the base combinations at position +1 on cleavage scores. Variants with base pairs at all but position +1 were included in this analysis.
FIG. 3a shows a structural model of human DICER in a dicing state. The dsRNA was modeled into the cryo-EM structure of human DICER, based on the crystal structure of dsRNA-bound Aa RNase III. DICER dsRBD was then superimposed with that of Aa RNase III to predict its position in a dicing state.
FIG. 3b shows Pre-miRNAs used in the massively parallel assay in which the 5-bp and 3-bp windows (positions -1 to 3, -1 to +1, and +1 to +3 relative to the starting position of 3p miRNA) were targeted for randomization based on the structural model.
FIG. 3c shows SDS-PAGE results of purified proteins.
FIG. 3d shows size-exclusion chromatograms of purified proteins.
FIGS. 4a and 4b show the distribution of read counts of variants.
FIGS. 4c and 4d show the distribution of cleavage scores of variants.
FIGS. 4e to 4h show correlations of cleavage scores of variants between different conditions of varying reaction time.
FIG. 4i shows the distribution of cleavage scores for variants measured after randomizing the region from +1 to +3 positions.
FIGS. 5a and 5b illustrate the structural impact on cleavage scores at position +1. G-U pair was considered as a mismatch only when located between mismatches. (p, pair; m, mismatch).
FIGS. 5c and 5d show the impact of the base combinations at +1 position on cleavage scores. Variants with base-pairs at all but position +1 were included in this analysis.
FIG. 6a shows analysis results of in vitro processing of pre-let-7a-1 variants by human DICER. RNA substrates each vary in their sequences at positions -1 to +1 (top panel). Pre-let-7a-1 variants were processed by human DICER and resolved on a urea-polyacrylamide gel (bottom left panel). Relative cleavage was calculated by quantifying the band intensity (1-uncleaved/input) (bottom right panel). Bars indicate mean±s.d. (n=3, independent experiments). Statistical significance was determined using Student's two-tailed t-test compared to GCm. **P < 0.01, ***P < 0.001. (WT, wild-type)
FIG. 6b illustrates the dsRNA processing experiment using pre-miRNA-like duplexes with relocated GYM motifs and human DICER. Each pre-miRNA duplex differs in the sequence from positions -1 to +2 (top panel). The duplex variants were processed by human DICER and dissolved in urea-polyacrylamide gel (bottom panel). The cleavage products and corresponding cleavage sites are indicated by arrowheads.
FIG. 6c shows the results of in vitro processing analysis of pre-let-7a-1 variants by Drosophila Dcr-1. Bars represent the mean ± standard deviation (n = 3, independent experiments). Statistical significance was determined using Student's two-tailed t-test compared to GCm. **P < 0.01, ***P < 0.001. (WT, wild-type)
FIG. 6d illustrates the dsRNA processing experiment using pre-miRNA-like duplexes with relocated GYM motifs and Drosophila Dcr-1.
FIG. 6e shows the dsRNA processing experiment using RNA duplexes with 3' overhangs of various nucleotide lengths (1-nt to 3-nt 3'). The duplex ends were mismatched so that the 5' end could bind to the 5' pocket. The duplexes were processed by DICER and dissolved in urea-polyacrylamide gel. The products from 5' counting, 3' counting, and GYM motifs are indicated by white, gray, and black arrowheads, respectively.
FIG. 6f shows a structural model of human DICER in the dicing state.
FIG. 6g shows the in vitro processing of pre-let-7a-1 variants by DICER(ΔdsRBD). Bars represent the mean ± standard deviation (n = 3, independent experiments). Statistical significance was determined using Student's two-tailed t-test compared to GCm. ***P < 0.001. (NS, not significant)
FIG. 6h shows the in vitro processing of duplexes by DICER(ΔdsRBD) or DICER(AA) mutants. The duplexes differ in the sequence from positions -1 to +2 (left panel). The duplexes were processed by DICER and dissolved in urea-polyacrylamide gel (right panel).
FIG. 7a shows the results of in vitro processing analysis by human DICER using pre-let-7a-1 with various GYM motifs. The 5' end of the substrate was radiolabeled. (†, nicked product at the 3p position)
FIG. 7b illustrates the proportion of processing by DICER over time in an in vitro analysis using pre-let-7a-1 with various GYM motifs. The 5' end of the substrate was radiolabeled. Squares represent the mean values (n = 2, independent experiments).
FIG. 7c shows the results of in vitro processing analysis in the presence of TRBP. The 5' end of the substrate was radiolabeled. Bars represent the mean ± standard deviation (n = 3, independent experiments). Statistical significance was determined using Student's two-tailed t-test compared to GCm. ***P < 0.001. (†, nicked product at the 3p position)

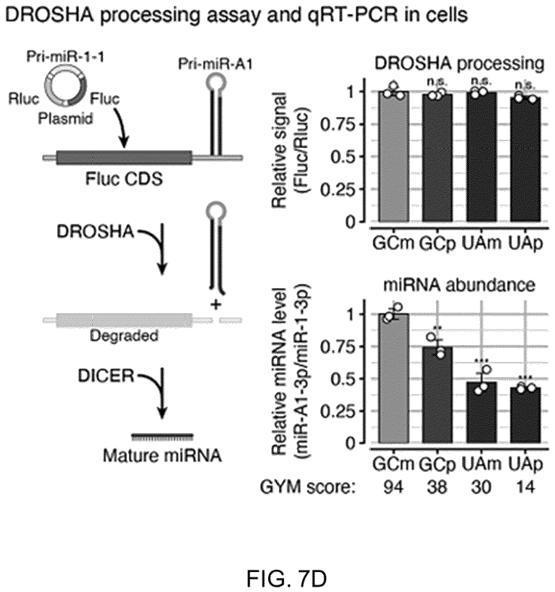

FIG. 7d shows the analysis of DROSHA processing of pre-miR-A1 variants and measurement of miRNA levels in HEK293T cells. The left panel provides a schematic overview of the experiment. The top right panel shows the luciferase assay. The firefly luciferase signal was normalized to the Renilla luciferase (Rluc) signal. The bottom right panel shows miRNA levels measured by qRT-PCR. TaqMan probes were designed to target the common sequence of the variants. Bars represent the mean ± standard deviation (n = 3, biological replicates). Statistical significance was determined using Student's two-tailed t-test compared to GCm. **P < 0.01, ***P < 0.001.
FIGS. 7e and 7g show the processing of duplex variants by human DICER. The cleavage products and corresponding cleavage sites are indicated by arrows. (*, radiolabeled 5' phosphate)
FIG. 7f shows the results of in vitro processing analysis by Drosophila Dcr-1 using pre-let-7a-1 with various GYM motifs. The 5' end of the substrate was radiolabeled. (†, nicked product at the 3p position)
FIG. 7g shows that the duplex had a base-pair at its terminus (marked in shade) so that the 5' end cannot be incorporated into the 5' pocket.
FIG. 8a shows the in vitro processing of pre-let-7a-1 variants by human DICER ΔdsRBD.
FIG. 8b shows the amino acid sequence alignment of dsRBDs of metazoan DICERs.
FIG. 8c shows the in vitro processing of duplex variants by human DICER point mutants at the indicated positions. The cleavage products and corresponding cleavage sites are indicated by arrows. (*, radiolabeled 5' phosphate)
FIGS. 8d and 8e show the in vitro processing of prelet-7a-1 variants by DICER R1855L (d) or R1855A/E1859A (AA) (e). Bars represent the mean (n = 2, independent experiments) (d) or the mean ± standard deviation (n = 3, independent experiments) (e). Statistical significance was determined using Student's two-tailed t-test compared to GCm. **P < 0.05, ***P < 0.001. (†, nicked product at the 3p position)
FIG. 8f shows the in vitro processing of duplex variants by DICER AA mutants. The cleavage products and corresponding cleavage sites indicated by arrows are minimally affected by the GYM motif variants, contrasting with the results of WT DICER shown in FIGS. 6b and 6d.
FIG. 9a provides a schematic overview of the DICER structural experiments (n = 2, biological replicates).
FIG. 9b shows the results of miRNA quantification. Spike-ins were used for normalization. (RPM, reads per million)
FIG. 9c illustrates the in vitro processing of pre-miR-27b variants by DICER wild-type (WT) or dsRBD mutants. The RNA substrates, radiolabeled at the 5' end, differ in sequence at positions -1 to +1 and have different GYM scores (top panel). Relative cleavage was calculated by quantifying band intensity (1 - uncleaved/input) (bottom panel). Bars represent the mean ± standard deviation (n = 3, independent experiments). Statistical significance was determined using Student's two-tailed t-test compared to the WT substrate. ***P < 0.001.
FIG. 9d compares cleavage accuracy (left panel). For a given miRNA, the most abundant 5'-isomiR was identified in the WT sample. Cleavage accuracy was estimated by quantifying the fold change in the proportion of the most abundant 5'-isomiR. The bar graph shows the number of miRNAs for which the major 5'-isomiR was significantly affected by the mutation. (Student's two-tailed t-test, P < 0.01) (right panel)
FIG. 9e shows a schematic (left panel) for the analysis of GYM motif conservation in pre-miRNAs and the distribution of GYM motifs at the indicated positions in natural pre-miRNAs across species (right panel). (C. *elegans, Caenorhabditis elegans;* C. *briggsae, Caenorhabditis briggsae; P. pacificus, Pristionchus pacificus*.)
FIG. 9f illustrates the correlation between GYM scores and alternative processing. The analysis was performed using miRNAs registered in miRGeneDB. For a given miRNA, if the proportion of the most abundant 5'-isomiR was 80% or more, it was classified into the homogeneous processing group (n = 203); otherwise, it was classified into the alternative processing group (n = 76).
FIG. 10a compares miRNA expression in HCT116 cells. (RPM, reads per million)
FIG. 10b compares cleavage accuracy in HCT116 cells. For a given miRNA, the most abundant 5'-isomiR was identified in the WT sample, and the fold change in its proportion in each sample was measured as cleavage accuracy. The bar graph shows the number of miRNAs for which the major 5'-isomiR was significantly affected by the mutation. (Student's two-tailed t-test, P < 0.01)
FIG. 10c compares miRNA expression in HEK293T cells. (RPM, reads per million)
FIG. 10d compares cleavage accuracy in HEK293T cells. For a given miRNA, the most abundant 5'-isomiR was identified in the WT sample, and the fold change in its proportion in each sample was measured as cleavage accuracy. The bar graph shows the number of miRNAs for which the major 5'-isomiR was significantly affected by the mutation. (Student's two-tailed t-test, P < 0.01)
FIGS. 11a to 11c show the in vitro processing of variants of pre-miR-27b (a), pre-miR-21 (b), and pre-let-7d/f-1/i (c) by DICER WT or ΔdsRBD. The 5' end of the pre-miRNA was radiolabeled. The reactions were performed at different time points as indicated. Bars represent the mean ± standard deviation (n = 3, independent experiments). Statistical significance was determined using Student's two-tailed t-test compared to the WT substrate. *P < 0.05, **P < 0.01, ***P < 0.001. (†, nicked product at the 3p position)
FIGS. 12a and 12b illustrate examples of miRNAs whose DICER cleavage sites are affected by mutations in DICER dsRBD. The cleavage sites were inferred using the 5' ends of miRNAs in DICER-knockout HCT116 cells reconstituted with the indicated DICER. Annotations from miRBase release 21 were used as a reference. The cleavage sites and their positions are indicated by arrows. (RPM, reads per million)
FIGS. 13a and 13c show the results from DICER structural experiments in DICER-knockout HCT116 cells using the 5' end of miR-34a-3p (a) or the 3' end of let-7e-5p and the 5' end of let-7e-3p (c). Annotations from miRBase Release 21 were used as a reference. The positions of the major cleavage sites are indicated by arrows. (RPM, reads per million)
FIGS. 13b and 13d show the in vitro processing of pre-miR-34a variants (b) or pre-let-7e variants (d) by DICER WT or ΔdsRBD. The 5' end of the pre-miRNA was radiolabeled. The major cleavage products and corresponding cleavage sites are indicated by arrows. Since DICER ΔdsRBD has reduced activity, the reactions were performed at different time points as indicated.
FIG. 13e shows the GYM scores at the -1 position of human pre-miRNAs. The analysis was performed using miRNAs registered in miRGeneDB (n = 383). The dotted line represents the average GYM scores at the surrounding positions (-2 and 0 positions).
FIG. 13f illustrates the alternative processing of pre-miR-9. The cleavage sites were inferred using the 5' end of miR-9-3p in DICER-knockout HCT116 cells rescued with DICER WT. The average proportions are indicated at the corresponding cleavage sites with arrows.
FIG. 13g shows the in vitro processing of pre-miR-9-1 by DICER. The GYM scores for each window (indicated by boxes) are shown. The 5' end of the pre-miRNA was radiolabeled. The major cleavage products and corresponding cleavage sites are indicated by arrows.
FIG. 14a provides the design of 3p-dominant shRNAs with optimal GYM motif (GUm), suboptimal GYM motif (CUp), and poor GYM score (CUp) GYM motifs. Sequence changes do not affect the mature siRNA sequence. The shRNA was encoded in a plasmid that also encodes pri-miR-1-1 under an independent promoter. 'Un' represents various numbers of uridines derived from the RNA polymerase III termination signal, and FLuc represents firefly luciferase.
FIG. 14b shows shRNA levels after 12 hours as measured by quantitative real-time PCR. The TaqMan probes were designed to target the same mature shRNA across variants. Bars represent the mean ± standard deviation (n = 3, biological replicates). Statistical significance was determined using Student's two-tailed t-test compared to GUm. ***P < 0.001.
FIG. 14c shows the results of the luciferase assay. The firefly luciferase signal was normalized to the Renilla luciferase (Rluc) signal. A control shRNA that does not target FLuc was used to measure gene silencing activity. Dots and bars represent the mean ± standard deviation (n = 3, biological replicates).
FIG. 14d provides the design of 3p-dominant DsiRNAs with various GYM motifs.
FIG. 14e shows the results of the luciferase assay. Dots and bars represent the mean ± standard deviation (n = 3, biological replicates).
FIG. 14f provides the design of 5p-dominant DsiRNAs with various GYM motifs.
FIG. 14g shows the results of the luciferase assay. Dots and bars represent the mean ± standard deviation (n = 3, biological replicates).
FIG. 14h shows the proposed model of Dicer processing.

### Best Mode for Carrying out the Invention

A better understanding of the present disclosure may be obtained through the following examples which are set forth to illustrate, but are not to be construed to limit, the present disclosure. It would be apparent to those skilled in the art that the scope of the present disclosure is not limited to these examples but is defined by the claims.

### EXAMPLES

(Throughout the specification, unless otherwise stated, the "%" used to indicate the concentration of a specific substance refers to (weight/weight) % for solid/solid, (weight/volume) % for solid/liquid, and (volume/volume) % for liquid/liquid.)

### EXAMPLE 1: Identification of a Sequence Motif That Promotes DICER Processing of dsRNA - Identification of "GYM Motif"

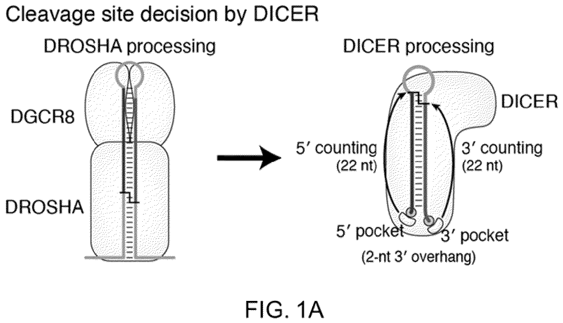

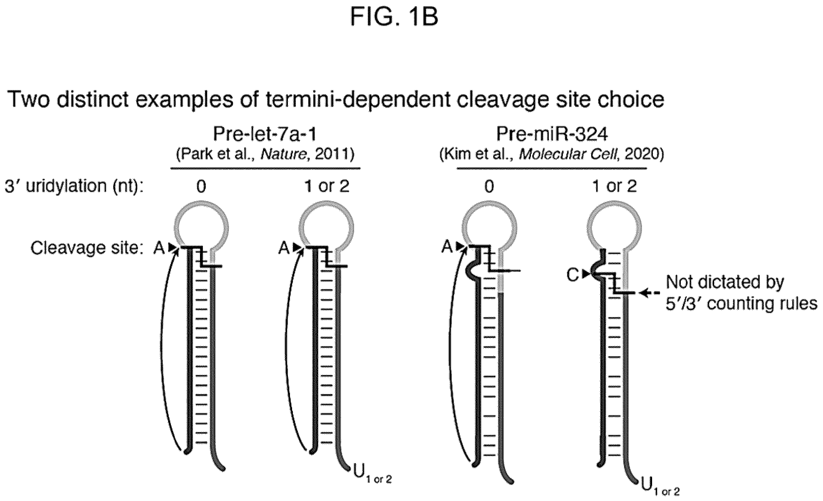

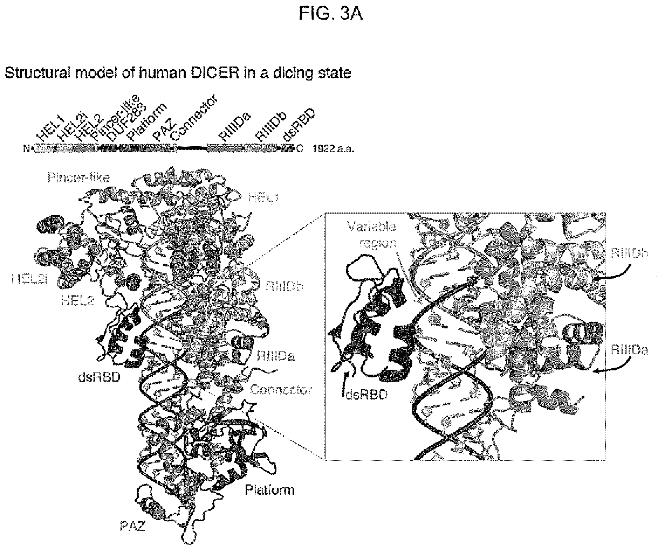

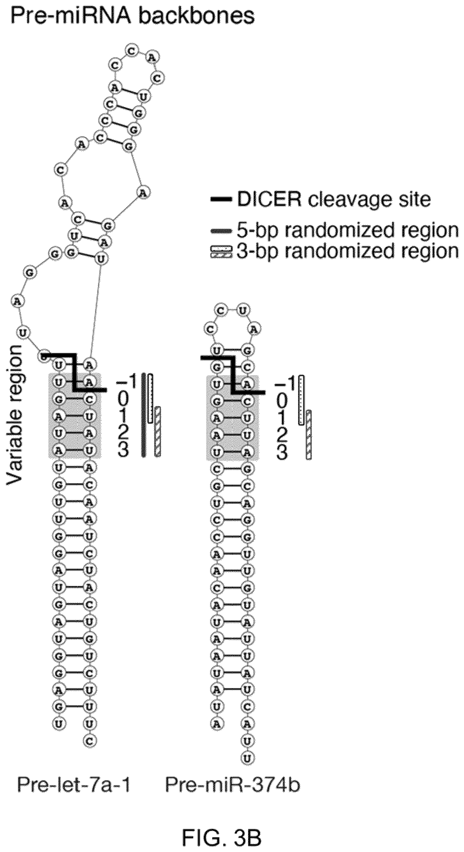

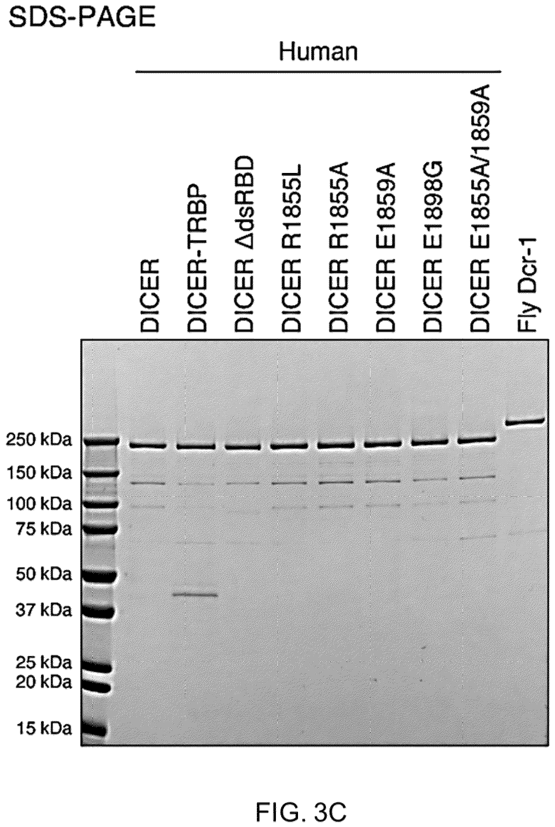

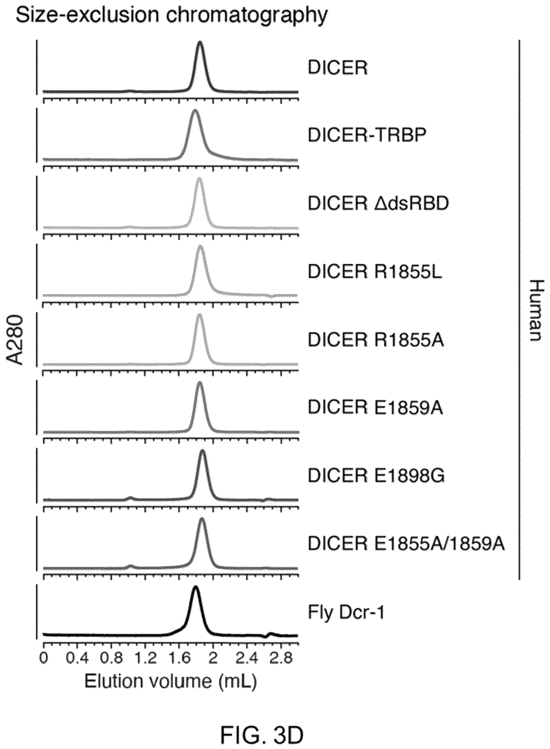

To comprehensively interrogate the upper stem region of the dsRNA, which is the substrate of DICER, a massively parallel assay that enables quantitative testing of a large number of variants was performed (FIG. 1a). In brief, a structural model was constructed and 1,048,576 pre-let-7a-1 variants for use as substrates of DICER were synthesized by randomizing the sequences within a 5-bp window in the upper stem (-1 to +3 relative to the cleavage site in the 3p strand) that is predicted to contact DICER in the structural model (FIGS. 1b, 3a, and 3b). After a brief incubation with purified human DICER protein (FIGS. 3c and 3d), 5% of the substrate pool was cleaved. The uncleaved RNA was gel-purified and sequenced using a method that allows for the efficient ligation of structured RNA (AQ-seq, accurate quantification by sequencing). The processing efficiency of DICER was quantified by dividing the fraction of each 3-bp motif in the input population (Fraction of input*_{variant i}*) by its fraction in the uncleaved pre-miRNA sample after the reaction (Fraction of uncleaved*_{variant i}*) (FIG. 1a), and this metric was termed the "cleavage score."

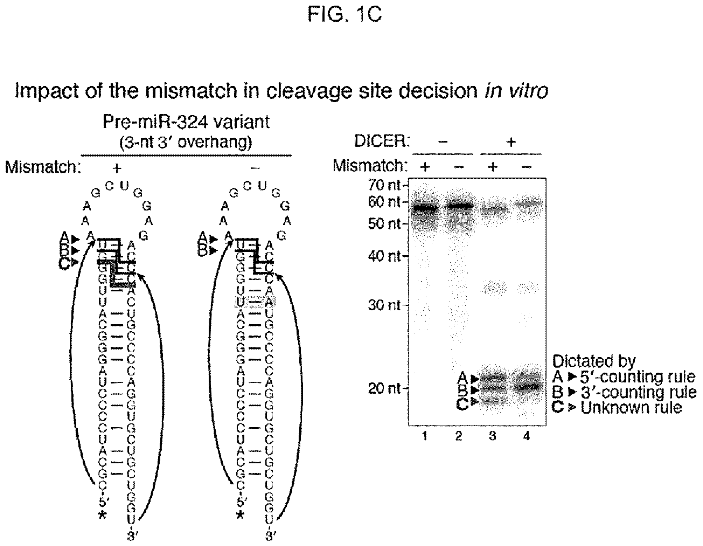

To identify sequences of the determinants for DICER processing, examination was made of structural and sequence characteristics of the pre-miRNA variants scoring within the top 0.1%. As expected, the top-scoring pre-miRNA variants showed an overall tendency for base-pairing throughout the randomized region (FIG. 1c, left panel). However, it was observed that a mismatch was enriched at position +1. Additionally, specific sequences were preferred within the randomized region (FIG. 1c, middle and right panels). Specifically, the 5p side of the -1 position was enriched in cytosine (C), while the 3p side was enriched in guanine (G), indicating a preference for the 5'-C-G-3' base pair. At the 0 position, the 5p side was enriched in G, and the 3p side in C, indicating a strong preference for the 5'-G-C-3' base pair. At the +1 position, G was depleted, and C was enriched on both the 5p and 3p strands, while sequence preference was less prominent at the +2 and +3 positions.

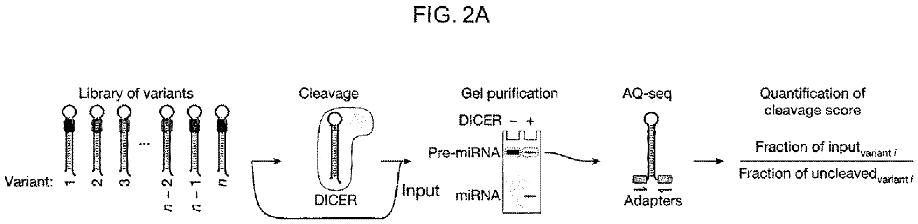

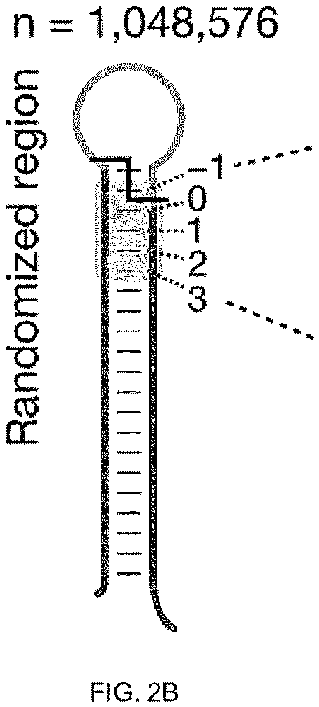

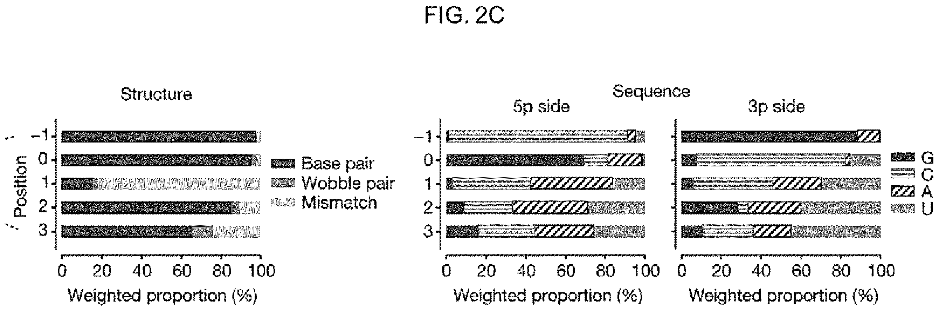

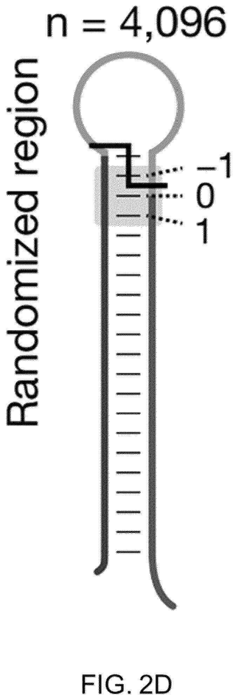

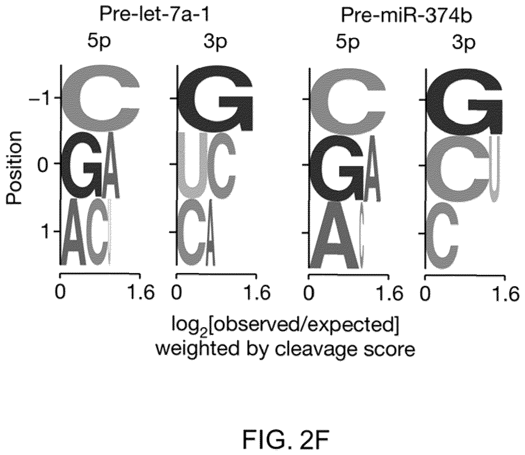

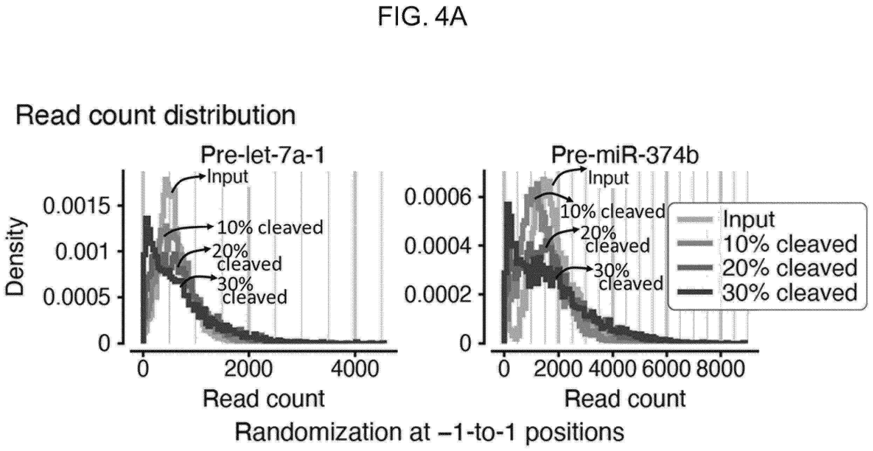

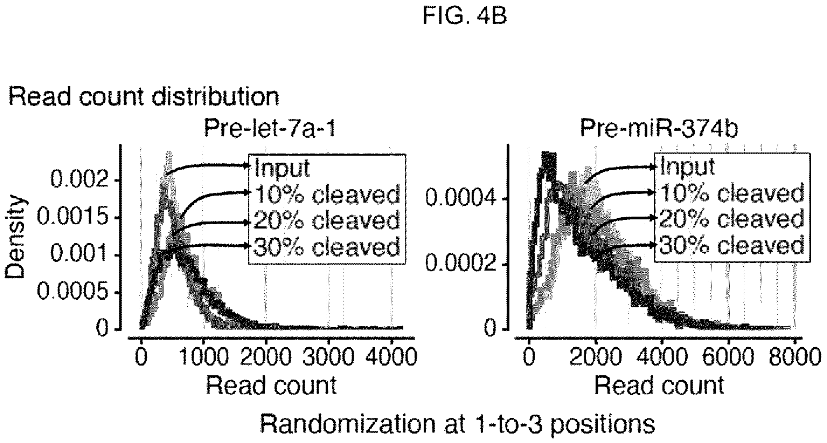

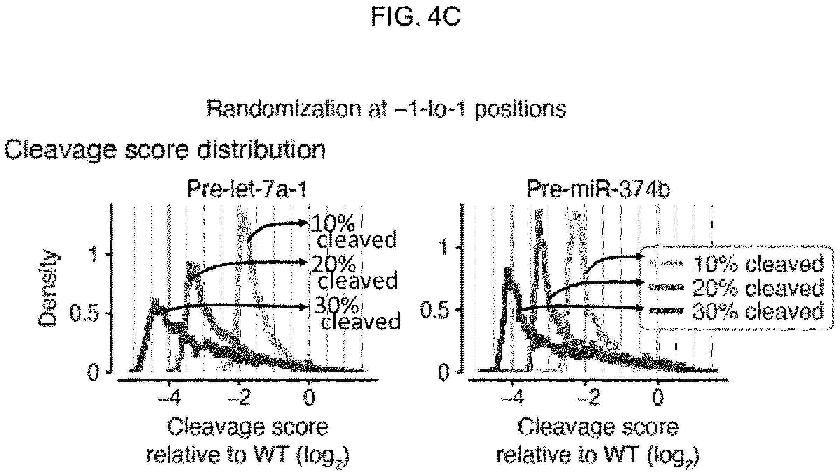

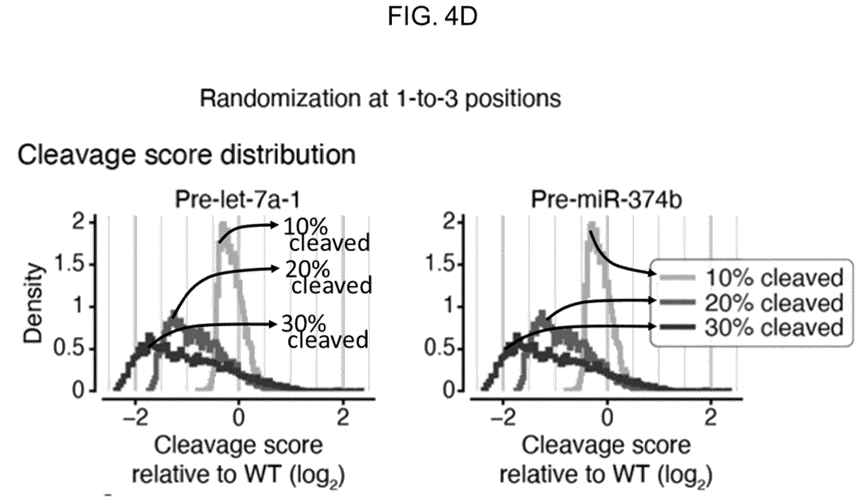

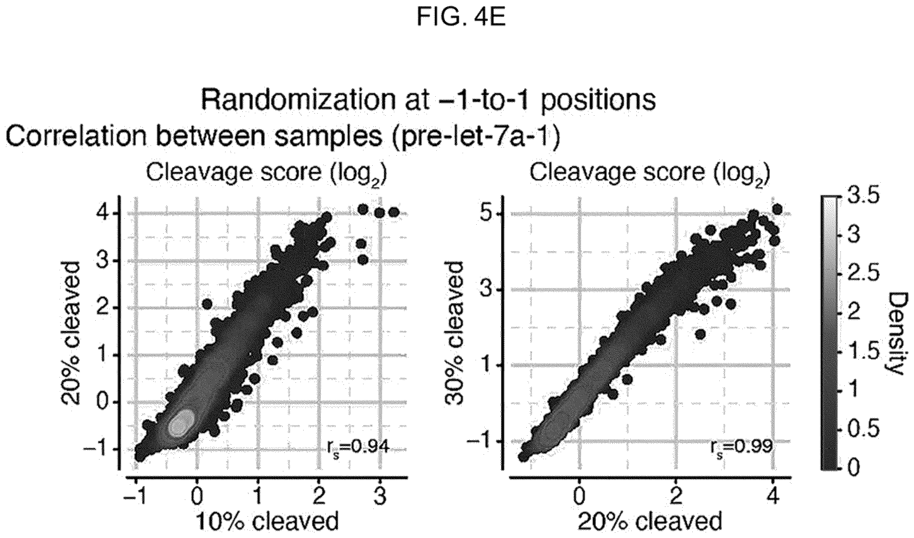

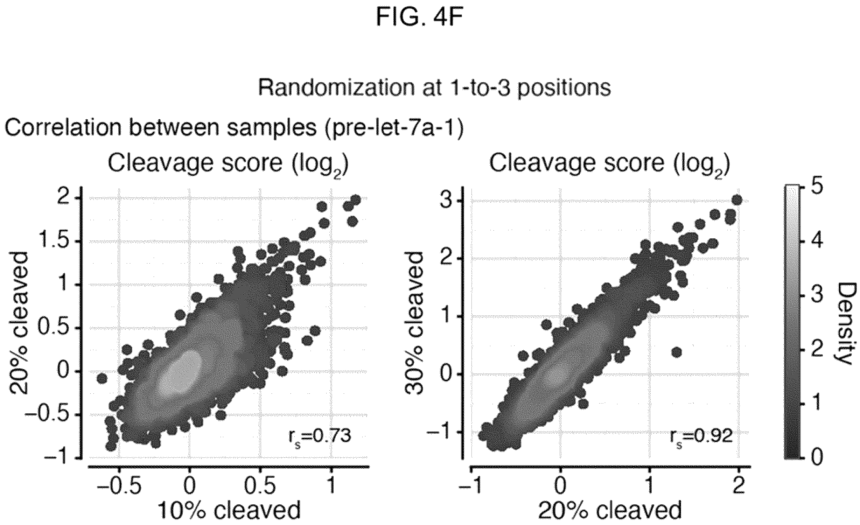

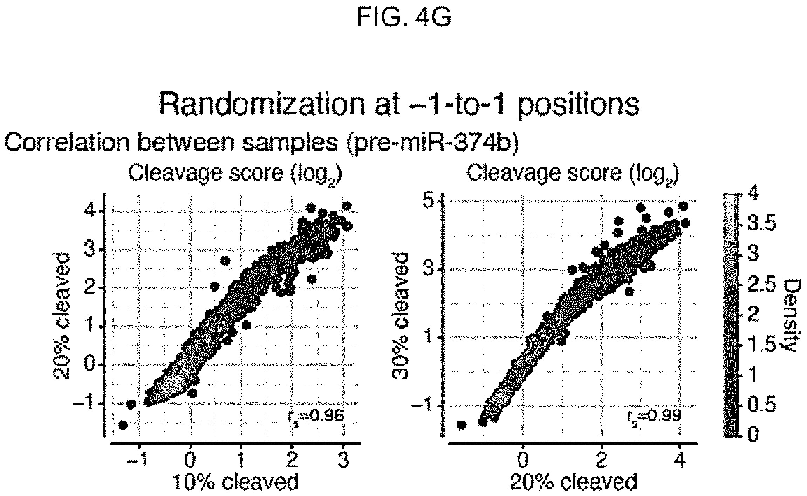

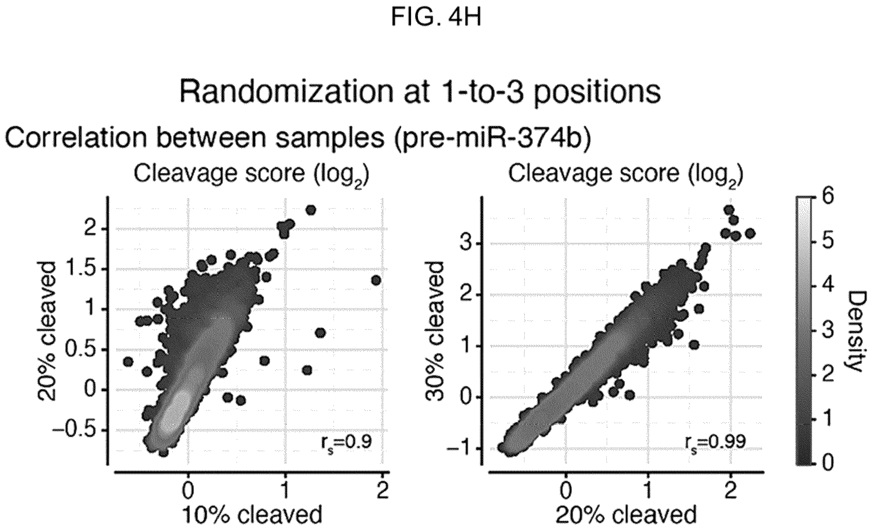

To increase the sequencing depth, a second round of massively parallel assays was conducted using pre-let-7a-1 with two 3-bp windows randomized (-1 to +1 position and +1 to +3 position) within the randomized 5-bp window region described above, to generate 4,096 variants per window (FIGS. 2d and 3b). Another relatively simple pre-miRNA backbone, pre-miR-374b, was also used to generate variants with the same two 3-bp windows randomized and used as DICER substrates (FIG. 3b). The cleavage reactions were performed briefly under conditions where 10%, 20%, or 30% of the substrate pool was cleaved (FIGS. 4a to 4d). Although the cleavage scores increased with incubation time, they showed strong correlations between conditions (FIGS. 4e to 4h), indicating that the experiments were within the dynamic range.

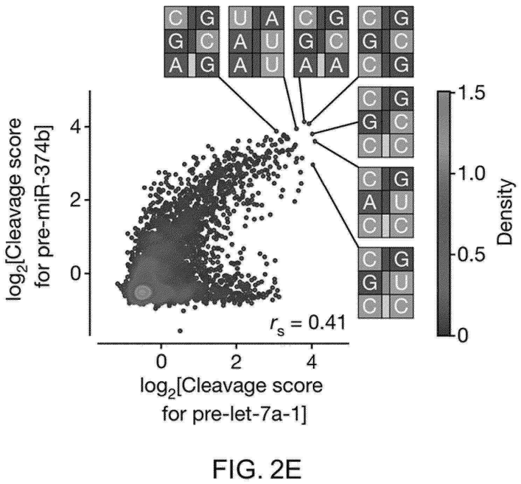

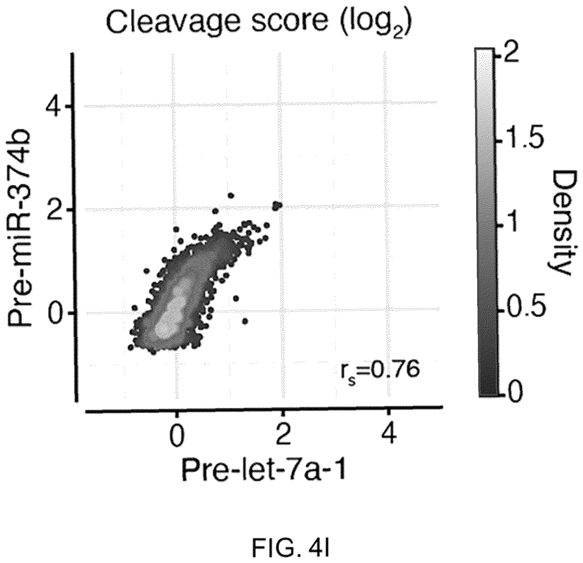

Variants randomized at the -1 to +1 positions showed a wide distribution of cleavage scores (FIG. 2e). The two different pre-miRNAs, pre-let-7a-1 and pre-miR-374b, showed a distinct correlation, particularly among the top-scoring sequences. These results suggest that the -1 to +1 position region plays a crucial role in regulating pre-miRNA processing independently of the backbone. Furthermore, when the region from +1 to +3 positions was randomized, the cleavage scores did not vary significantly between variants, indicating that the region from -1 to +1 positions has a greater impact on DICER processing than the region from +1 to +3 positions (FIG. 4i).

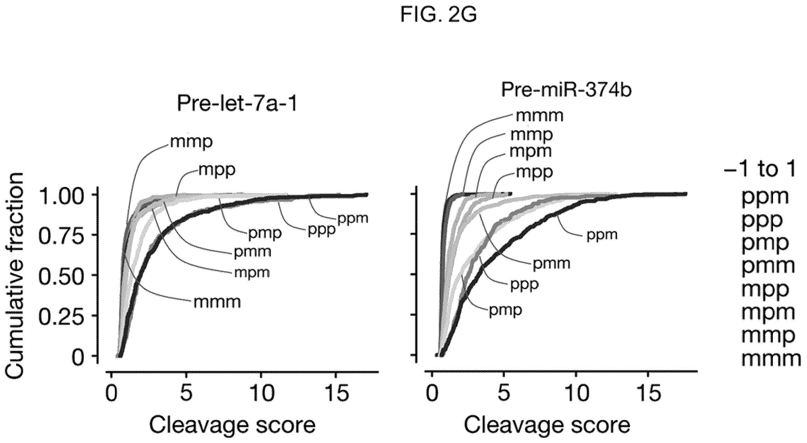

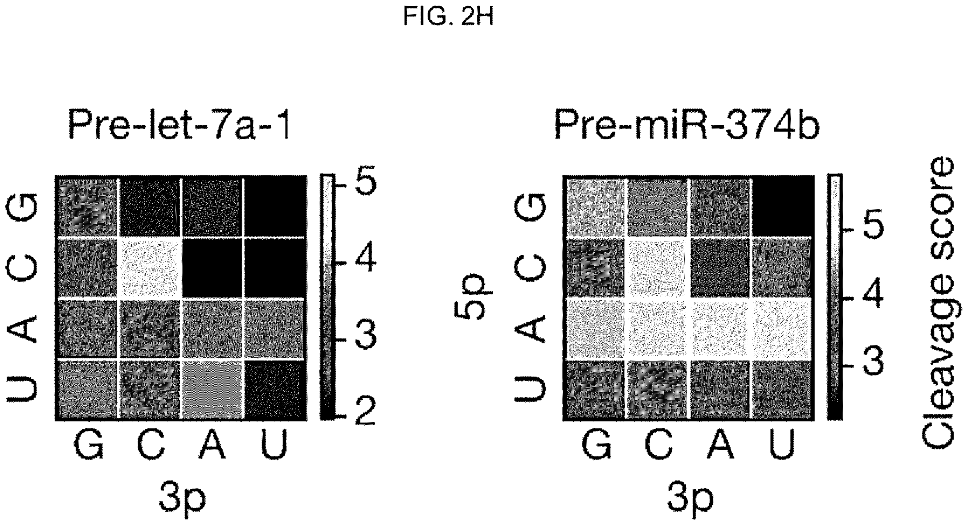

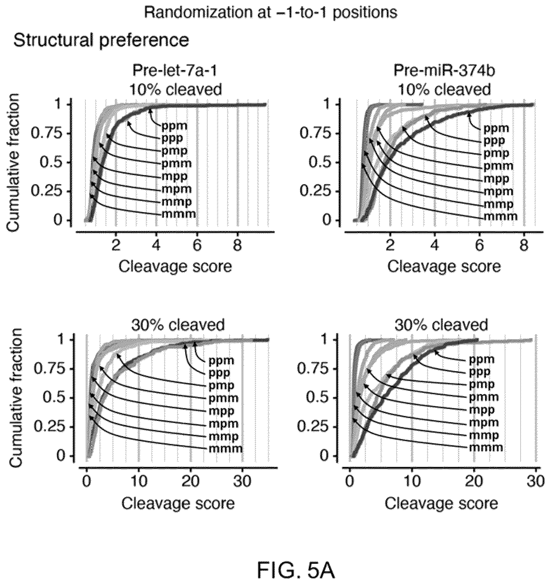

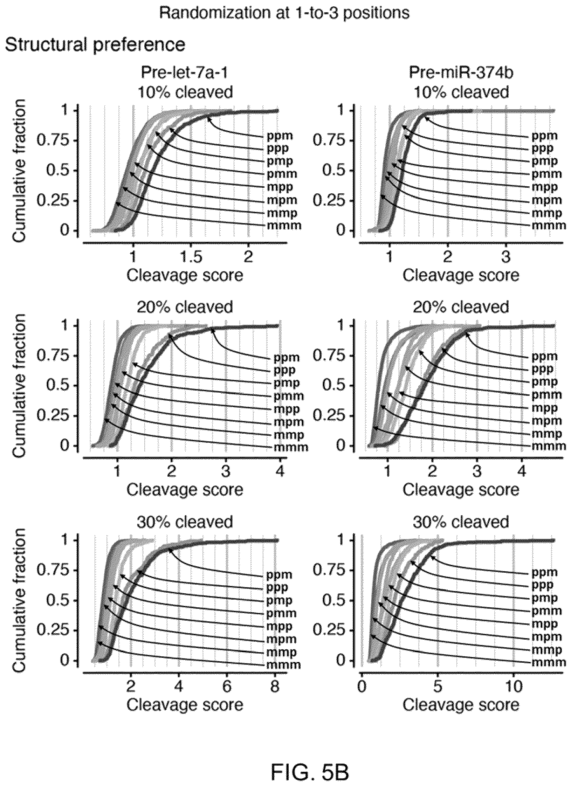

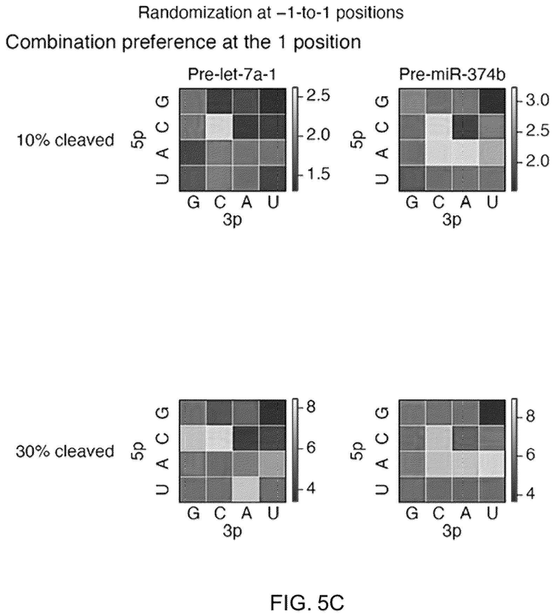

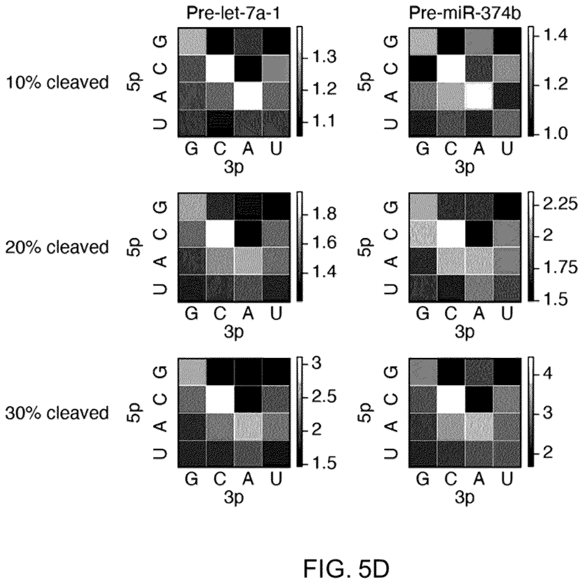

Additionally, as shown in FIGS. 2g, 5a, and 5b, while base pairing between the nucleotides of the 5p and 3p strands is generally favorable for processing, mismatches at the +1 position were associated with higher cleavage scores. It was found that certain base combinations were preferred at the +1 position, with the C-C mismatch generally exhibiting the strongest effect (FIGS. 2h, 5c, and 5d). These sequence and structural preferences at the +1 position were consistently observed across all tested conditions, regardless of sliding window, backbone, or reaction time.

In summary, the above experimental results allowed the inventors to identify a position-dependent 3-bp motif that strongly promotes DICER processing. This position-dependent 3-bp motif includes the following: a base-paired G at the -1 position on the 3p side, a base-paired pyrimidine (Y) at the 0 position on the 3p side, and a mismatched C or, less preferably, A (M) at the +1 position on the 3p side. Cleavage by DICER occurs between the G and Y bases. This element was named the "GYM motif" based on these bases. It is important to note that despite the clear consensus sequence of the GYM motif described above, the cleavage scores of the variants actually represent a continuum. Therefore, the GYM motif should be recognized as a quantitative characteristic based on cleavage scores rather than being defined dichotomously by the nucleotide sequence consensus. The inventors therefore devised the GYM score as an indicator to represent the strength of the motif. The GYM score for each 3-bp variant was assigned by calculating the average cleavage score of each 3-bp variant measured under conditions where 20% of the DICER substrates, pre-let-7a-1 and pre-miR-374b, were cleaved, and normalizing the average to a scale of 0-100.

### EXAMPLE 2: Verification of GYM Motif's Ability to Enhance DICER Processing of dsRNA (The GYM motif enhances dsRNA processing)

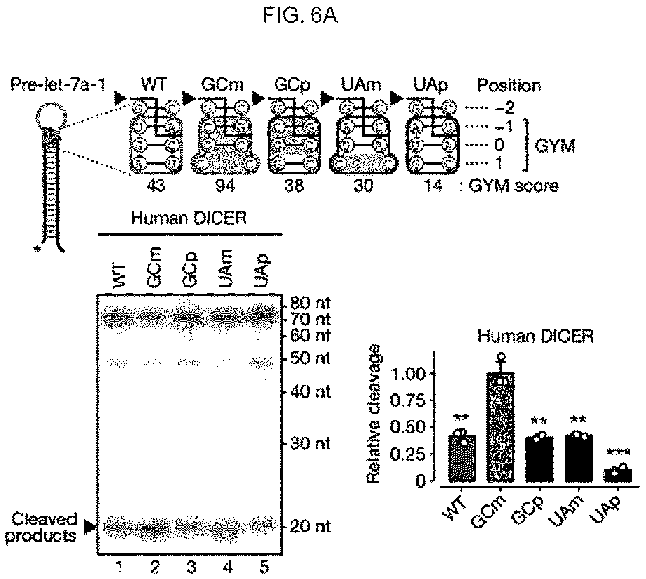

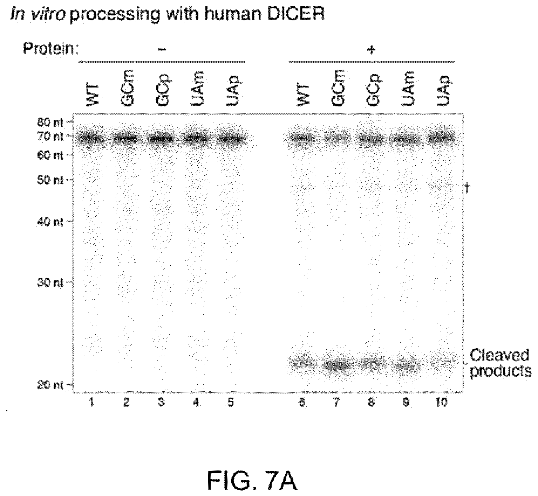

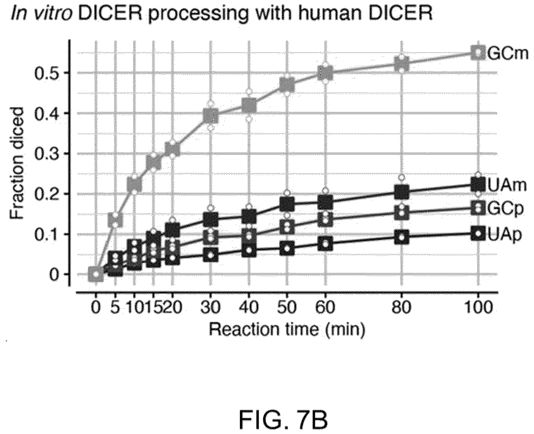

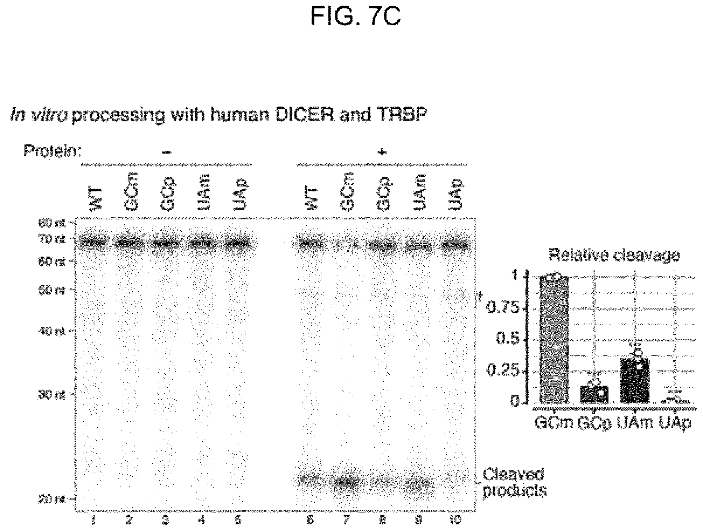

To validate the high-throughput data, an in vitro processing analysis was performed using pre-let-7a-1 with various GYM motifs (FIG. 6a). As a result, it was found that processing by DICER occurred with higher efficiency in a representative variant with a high GYM score (GCm, GYM score 94; m indicates a mismatch, same below) compared to the wild type (GYM score 43; FIGS. 6a, 7a, and 7b). Additionally, the variant with a 5'-G-C-3' pair at the +1 position (GCp, GYM score 38; p indicates that a base pair was formed at the corresponding position, same below) and the variant with a UA dinucleotide at the -1 and 0 positions (UAm, GYM score 30) were processed less efficiently than the aforementioned GCm variant. The variant with both regions replaced (UAp, GYM score 14) had the lowest processing efficiency. These results demonstrate that both the base-paired 'GY' and the mismatched 'M' parts are important for DICER processing and provide additional effects. A similar analysis was performed in the presence of TRBP, a dsRNA-binding protein that associates with DICER (FIG. 7c), confirming that DICER recognizes the GYM motif independently of TRBP.

Next, to examine the activity of the motif in cells, GYM variants were inserted into an artificial pri-miRNA hairpin embedded in the 3' untranslated region (UTR) of a luciferase reporter construct (FIG. 7d). This construct was used to monitor the activity of DROSHA by observing the reduction in luciferase expression when the hairpin was cleaved. As shown in FIG. 7d, the luciferase levels were not altered by the GYM motif, indicating that the GYM motif does not affect processing by DROSHA. In contrast, the levels of mature miRNA in the luciferase construct were correlated with the GYM score, consistent with the in vitro analysis results mentioned above, indicating that the GYM motif operates independently of the backbone.

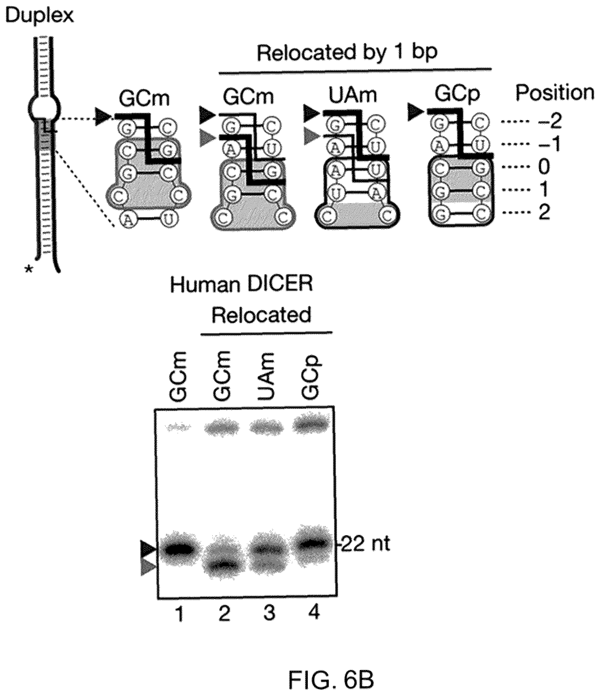

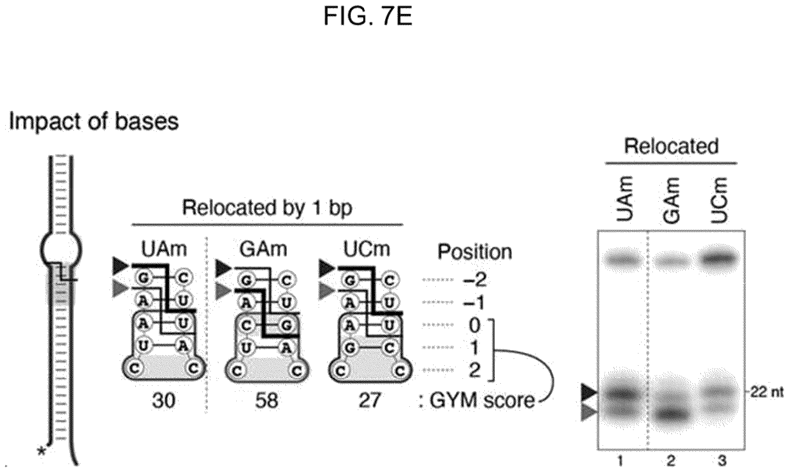

The contribution of the GYM motif to cleavage site selection was also examined. Specifically, pre-miRNA-like duplexes were designed with a high-scoring GYM motif properly positioned and with the motif shifted by 1 bp toward the terminus. As shown in FIG. 6b, the cleavage site also shifted by 1 bp due to the relocation of the motif. The motif lacking the GC dinucleotide at the -1 and 0 positions (UAm) also slightly influenced cleavage site selection. In contrast, the motif lacking a mismatch at the +1 position (GCp) did not significantly change the cleavage site. This indicates that the major influence of the GYM motif on cleavage site selection is exerted by the mismatch 'M' at the +1 position, while the GC dinucleotide at the -1 and 0 positions enhances the effect of the mismatch and increases processing efficiency (FIG. 7e). Collectively, these results demonstrate that the GYM motif not only promotes efficient processing but also acts as a key determinant of the cleavage site.

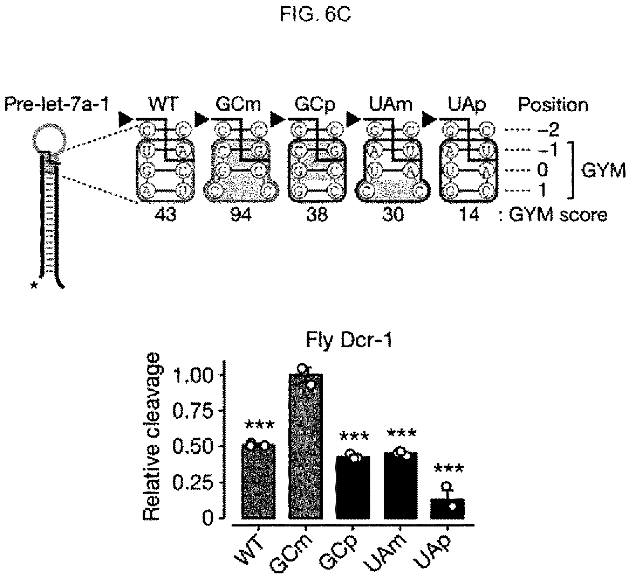

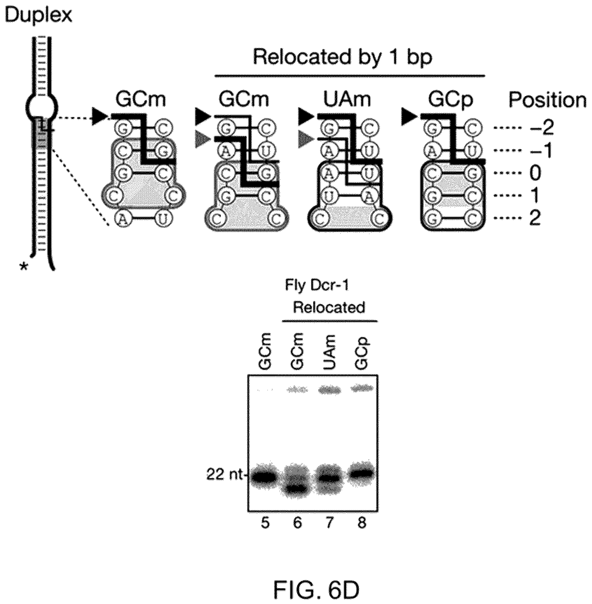

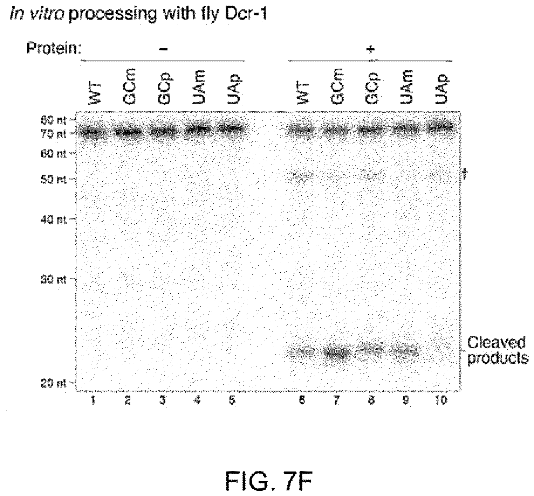

To gain further insights into the functional relevance of the GYM motif in other species, Drosophila Dicer-1 (Dcr-1) was investigated, and the results are shown in FIGS. 6c and 7f. Similar to human DICER, Drosophila Dcr-1 was found to prefer high-scoring GYM motifs. Additionally, when the position of the motif was changed, the cleavage site of Dcr-1 also shifted (FIG. 6d). This suggests that the GYM motif may play a highly conserved role in pre-miRNA processing in metazoans.

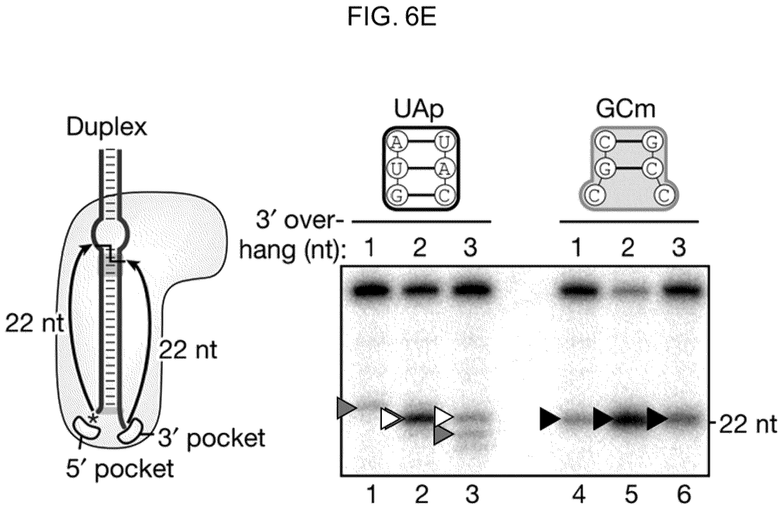

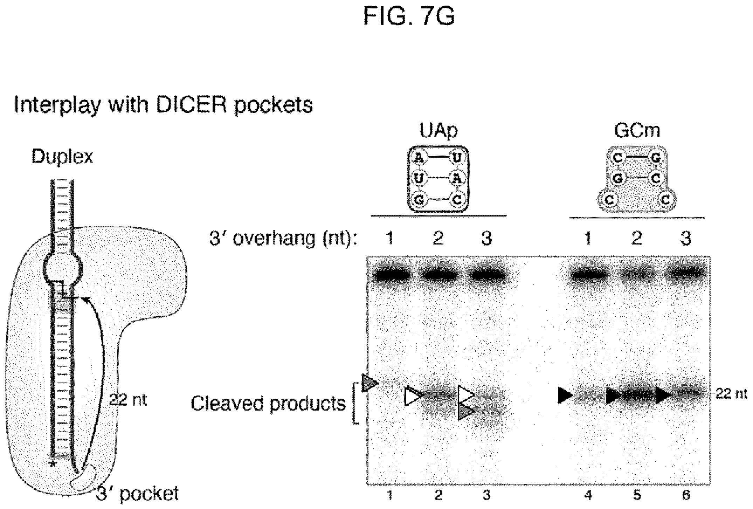

Since the current model of dsRNA processing assumes that DICER follows the 5' counting rule and the 3' counting rule, the interaction of the GYM motif with these terminal counting rules was investigated. To do so, RNA duplexes with 3' overhangs of varying nucleotide lengths (1-nt to 3-nt 3') were generated and tested. For the low-scoring GYM motif (UAp), a mixture of products generated by 5' counting and 3' counting was produced according to each overhang (FIG. 6e, white and gray arrows, respectively). For the high-scoring GYM motif (GCm), a single site was selected, resulting in uniform products regardless of the overhang length (FIG. 6e, black arrow). To promote 3' counting, RNA duplexes with terminal base pairs (indicated by shading in FIG. 7g) were also generated. In these RNA duplexes, the terminal base pairs prevent the 5' end from binding to the 5' pocket. Even in this case, similar to the results described above, the high-scoring GYM motif not only increased cleavage efficiency but also overrode the 3' terminal counting rule (FIG. 7g). Thus, it was confirmed that an optimal GYM motif is the dominant determinant of cleavage site selection and can override the 5' and 3' counting rules when in conflict.

### EXAMPLE 3: Mechanism of GYM Motif Recognition by DICER

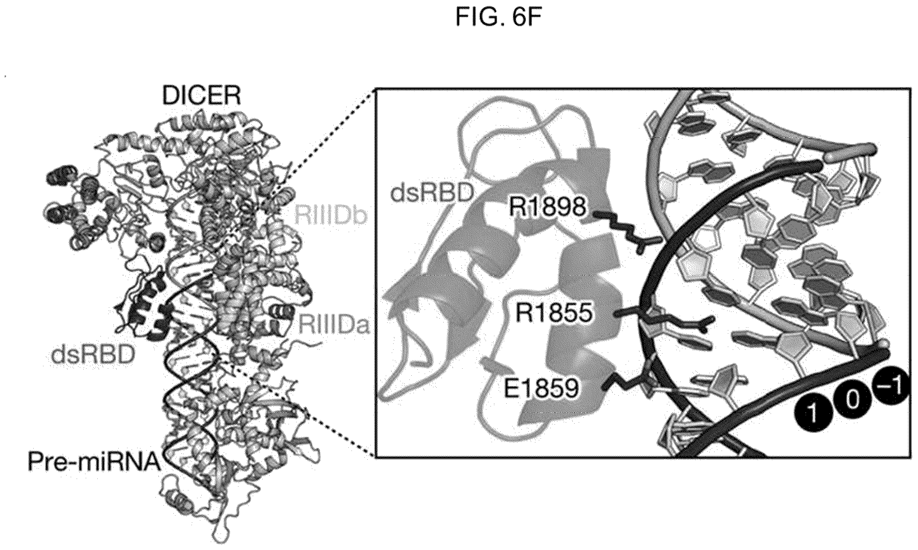

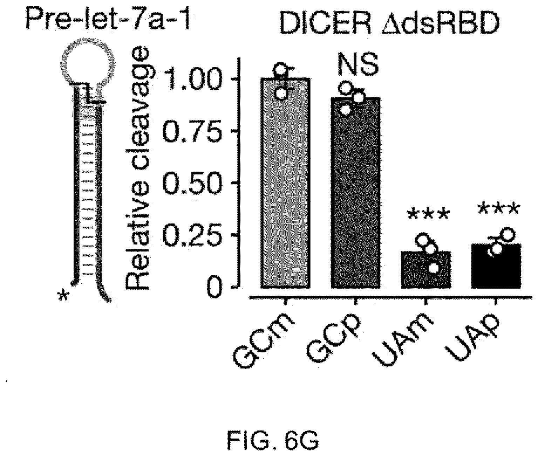

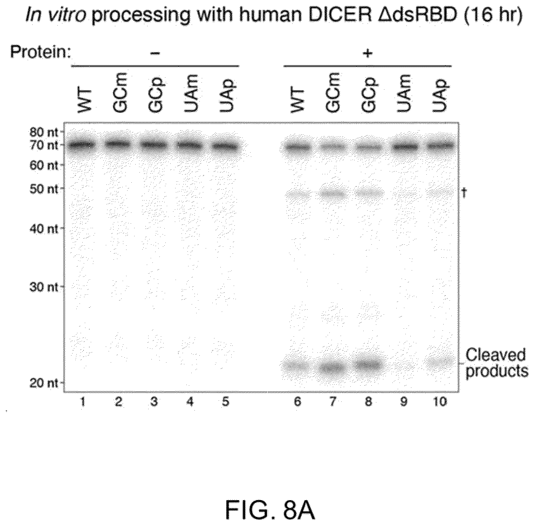

To investigate the mechanism of GYM motif recognition by DICER, a structural model of the DICER-dsRNA complex in the dicing state was constructed (FIG. 6f). The structural model utilized the pre-dicing structure of human DICER containing a pre-miRNA substrate (PDB: 5ZAL) and the structure of Aquifex aeolicus RNase III containing a dsRNA substrate (PDB: 2EZ6). In the structural model, the C-terminal dsRBD (double-stranded RNA-binding domain) was located near the GYM motif, suggesting its role in dsRBD function (FIG. 6f). Indeed, as shown in FIG. 6a, a DICER mutant lacking the dsRBD (ΔdsRBD) was unable to distinguish the mismatch at the +1 position. In contrast, the GC dinucleotide at the -1 and 0 positions was still preferred over the UA dinucleotide (FIGS. 6g and 8a). These results suggest that while the nucleotides at the -1 and/or 0 positions may be recognized by other domains, the dsRBD plays a crucial role in detecting the mismatch at the +1 position.

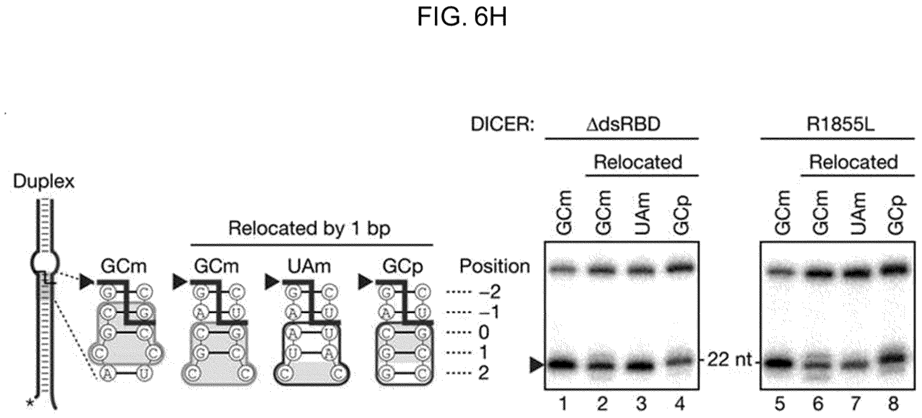

Additionally, when investigating cleavage site selection, as shown in FIG. 6h, unlike wild-type DICER (FIG. 6b), ΔdsRBD was no longer influenced by the position of the GYM motif, indicating that dsRBD affects the recognition of the GYM motif for cleavage site determination.

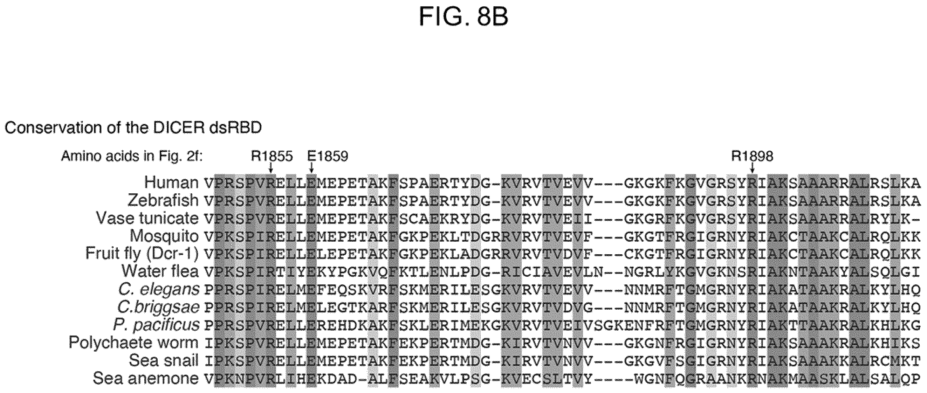

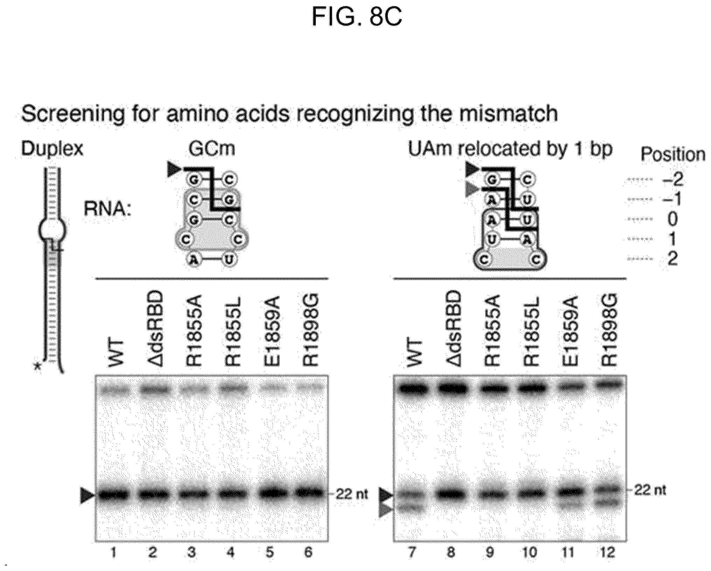

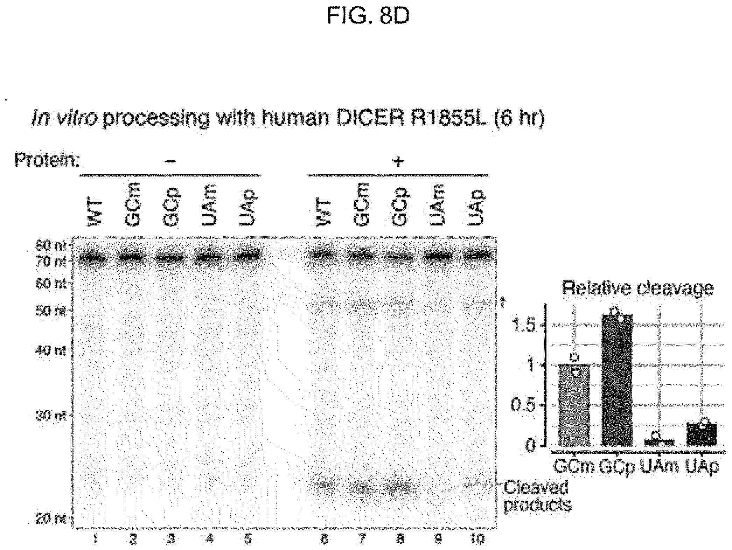

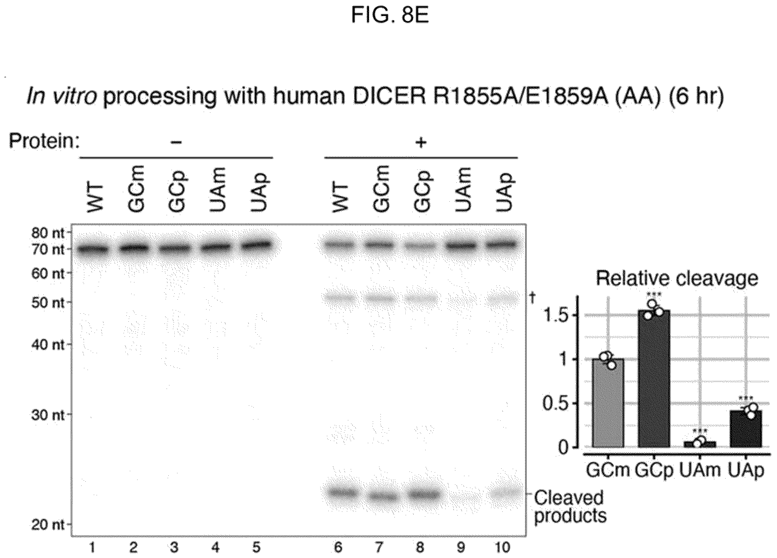

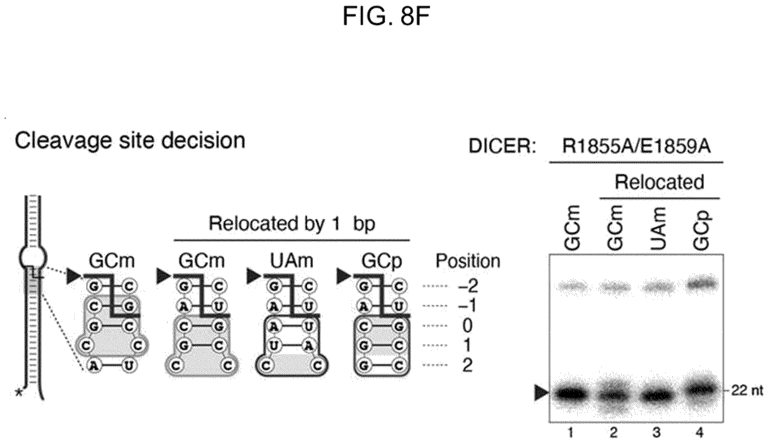

Based on these results, the dsRBD-dsRNA interface was closely examined to identify the residues responsible for motif recognition. A highly conserved arginine residue (R1855) in the first α-helix of dsRBD, which wraps around the minor groove of dsRNA, is positioned very close to the nucleotide at the +1 position (FIGS. 6f and 8b). This arginine residue, when substituted with leucine (R1855L), was found in cancer through The Cancer Genome Atlas, suggesting that this point mutation may cause defects in pre-miRNA processing. Indeed, as shown in FIG. 8c, substitution of arginine with leucine (R1855L) or alanine (R1855A) resulted in a reduced site-specific recognition of the mismatch within the GYM motif (FIG. 8c). Further examination of two other highly conserved residues near the mismatch at the +1 position of dsRNA, glutamate (E1859) and arginine (R1898), revealed that E1859 also contributes to motif recognition (FIGS. 6f, 8b, and 8c). The R1855L mutation and the double mutation (R1855A/E1859A, or "AA") lost the ability to preferentially recognize the mismatch at the +1 position (compare cleavage efficiency in FIGS. 6a and 8d, 8e; and compare cleavage site selection in FIGS. 6b, 6h, and 8f). Collectively, these results indicate that DICER primarily uses the highly conserved R1855 and E1859 residues of dsRBD to recognize the mismatch ('M') at the +1 position of the GYM motif, ensuring efficient and precise processing.

### EXAMPLE 4: Role of GYM Motif in miRNA Biogenesis

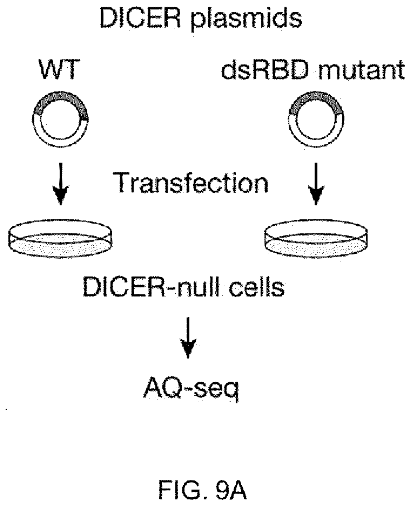

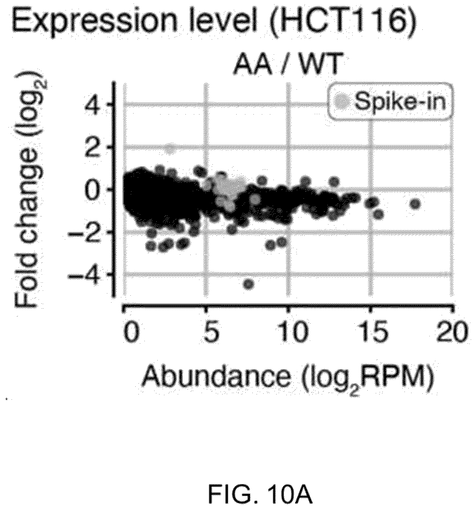

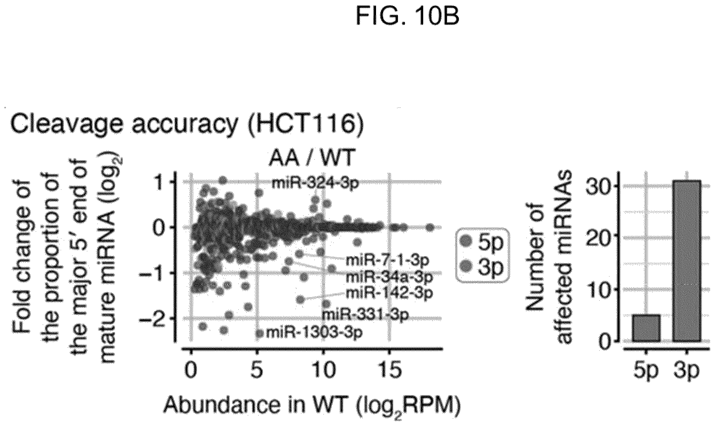

The biological relevance of the GYM motif in the maturation of endogenous miRNAs was analyzed. Wild-type or mutant DICER proteins were ectopically expressed in DICER-knockout HCT116 or HEK293T cells (FIG. 9a). Small RNAs were sequenced using the AQ-seq protocol to allow for bias-minimized quantification, enabling reliable comparisons between miRNA isomers (isomiRs) (FIGS. 10a to 10d).

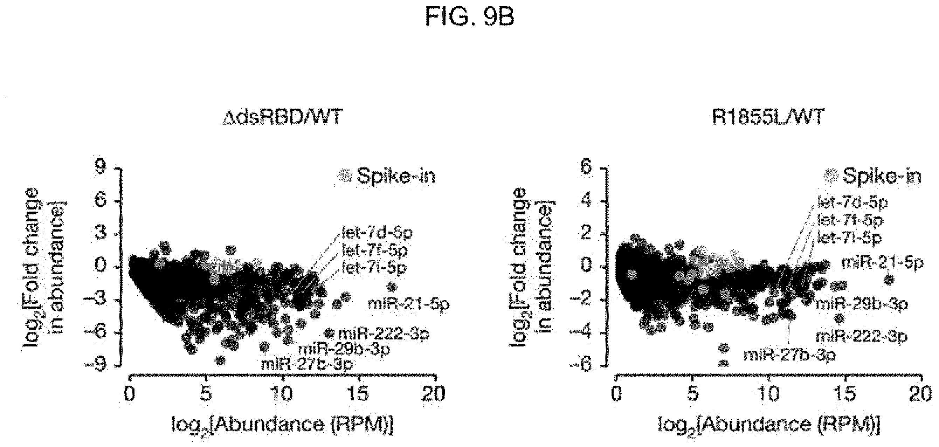

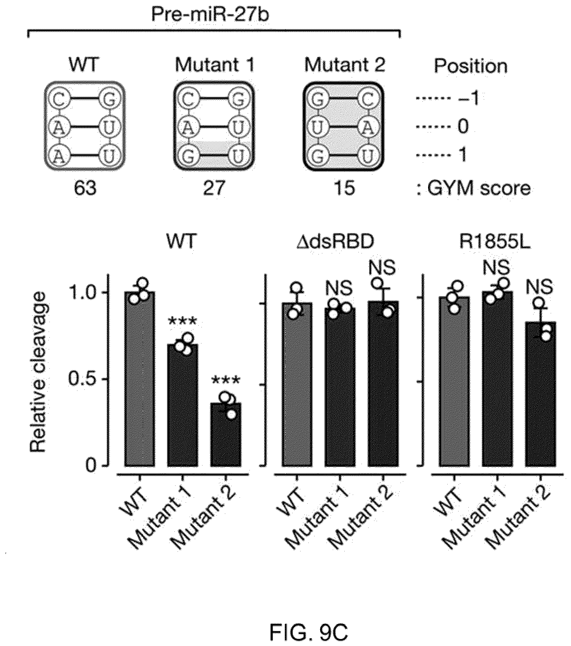

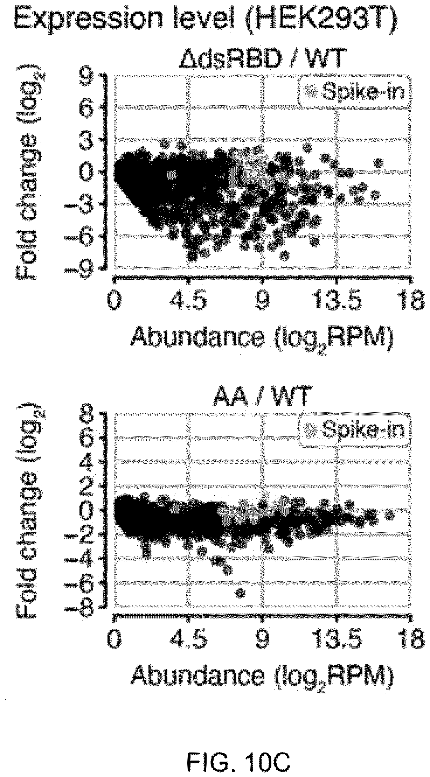

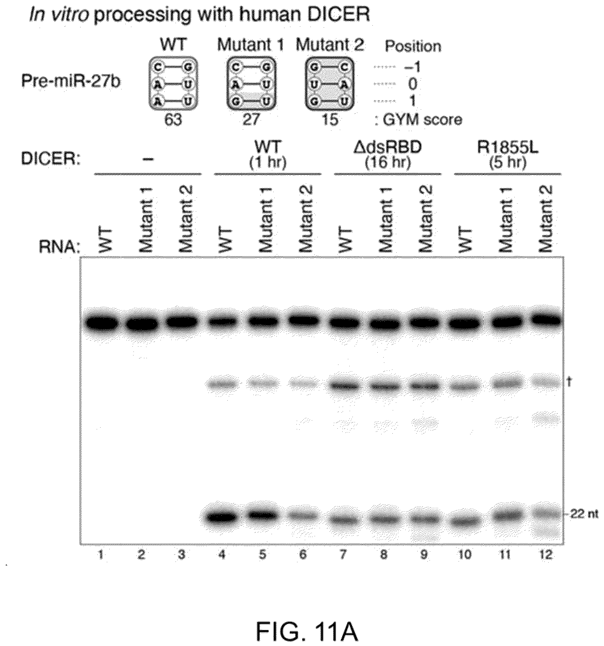

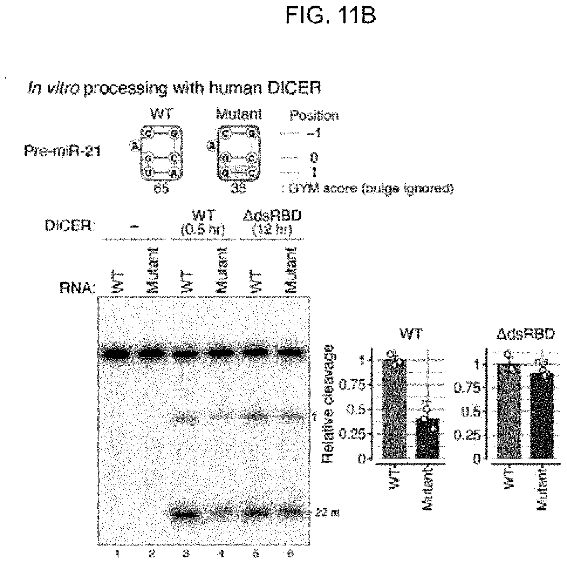

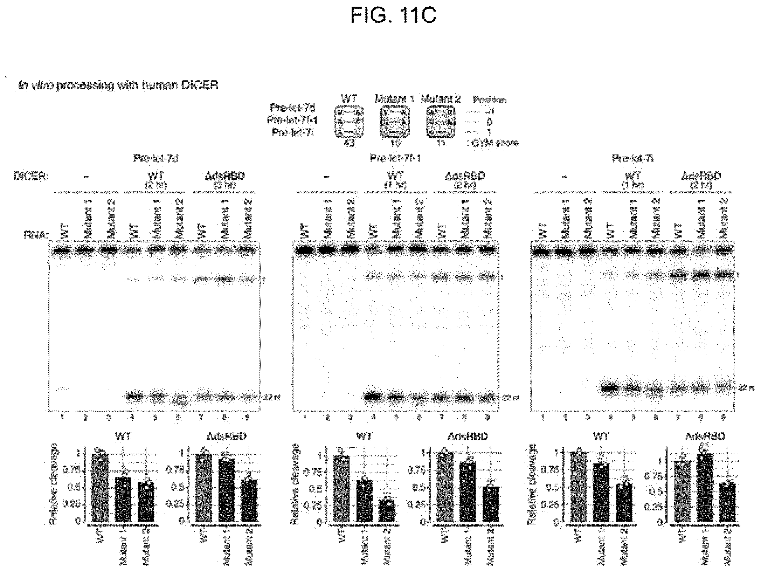

As a result, it was found that when dsRBD was deleted or the R1855 and E1859 residues of dsRBD were mutated, the amount of miRNA decreased (FIGS. 9b, 10a, and 10c). Since conserved and highly abundant miRNAs were strongly affected, it was investigated whether these miRNAs are generated in a GYM-dependent manner. As shown in FIGS. 9c and 11a to 11c, the mutations in dsRNA caused a reduction in DICER activity in vitro, requiring longer incubation times for pre-miR-27b, pre-miR21, pre-let-7d, pre-let-7f-1, and pre-let-7i. The processing efficiency decreased as the GYM motif of the pre-miRNA was weakened (lower GYM score), and this difference in processing efficiency diminished as mutations were introduced into dsRBD. Collectively, these findings suggest that the interaction between the GYM motif and DICER's dsRBD promotes miRNA processing by DICER both in cells and in vitro.

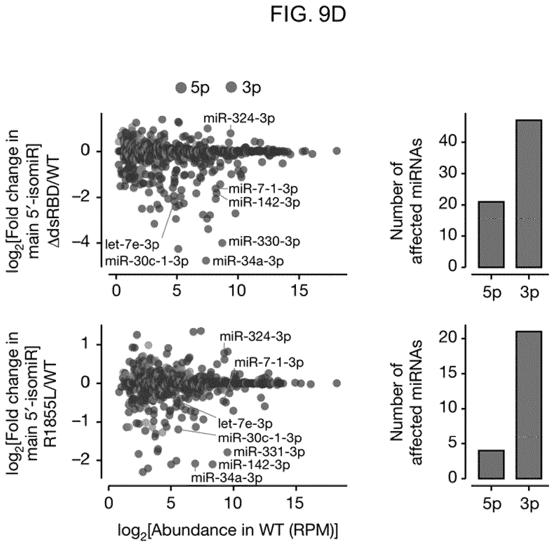

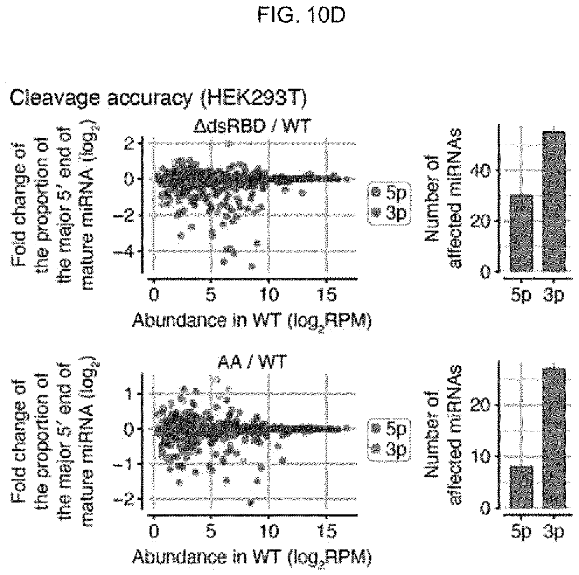

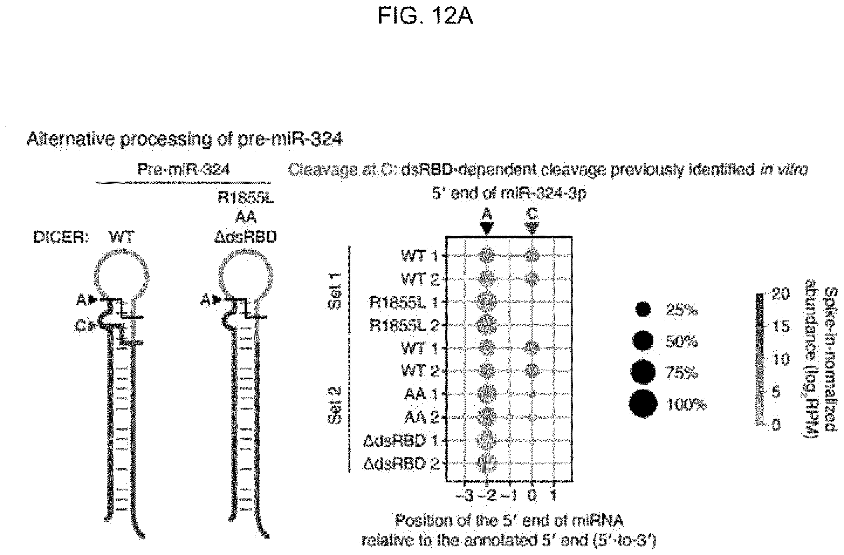

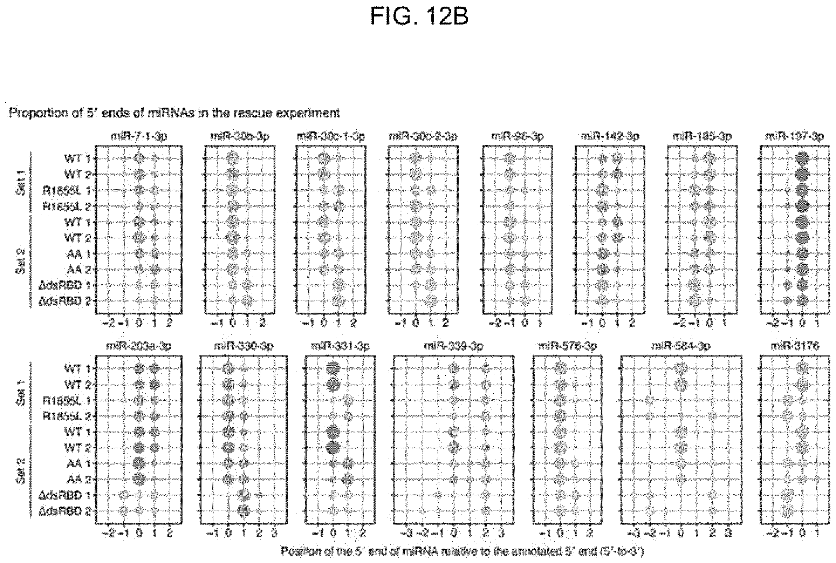

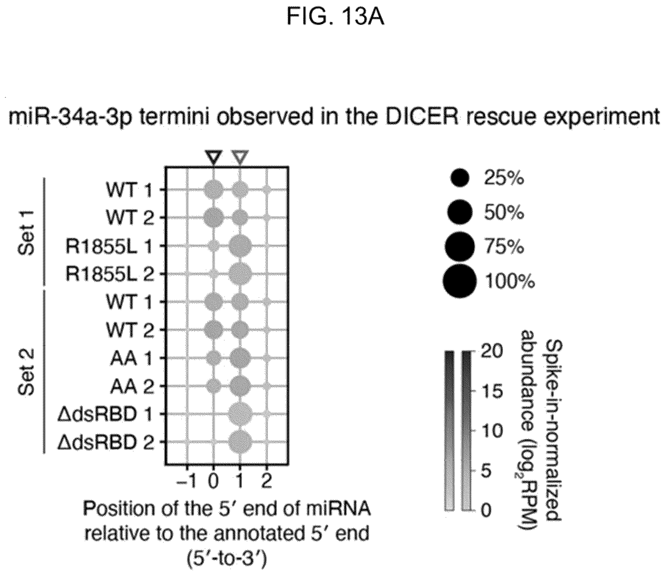

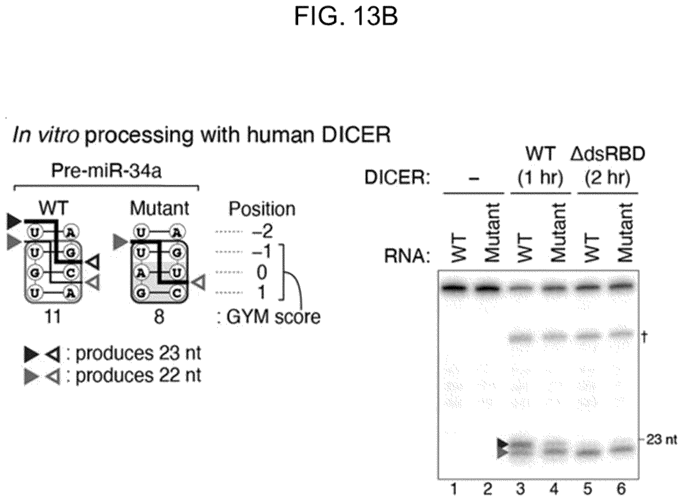

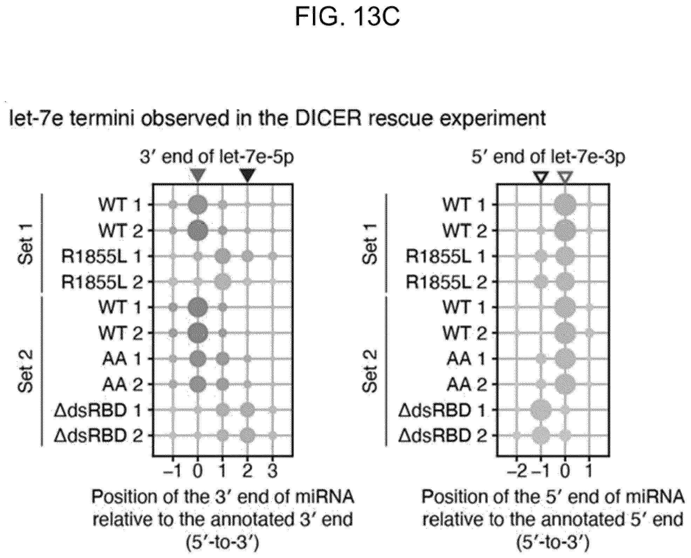

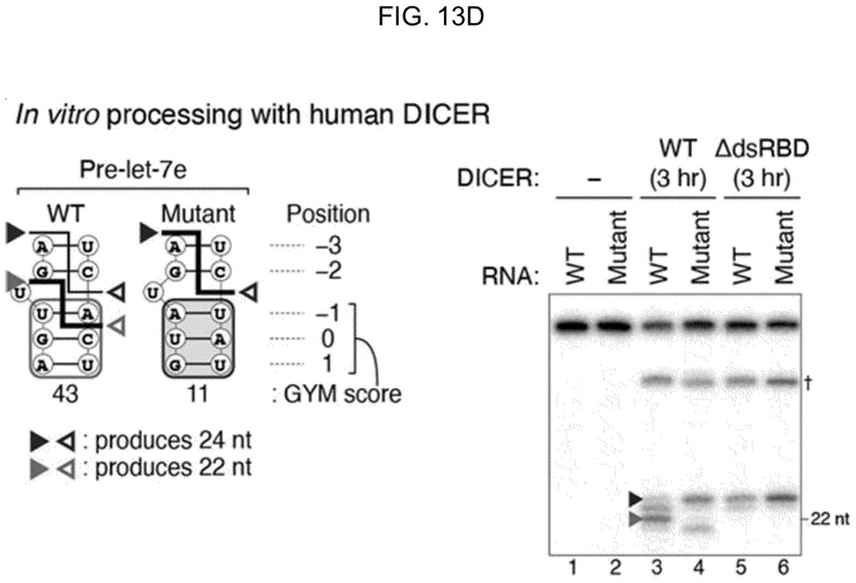

Additionally, as shown in FIG. 12a, changes in cleavage sites due to mutations in DICER's dsRBD were also observed. The most notable example was miR-324-3p, known to depend on DICER's dsRBD in vitro. Furthermore, we observed that many miRNAs were affected in their processing by mutations in dsRBD (FIGS. 9d and 12b). To infer the cleavage sites of DROSHA and DICER, the 5' ends of 5p miRNAs (FIG. 9d, blue dots) and 3p miRNAs (FIG. 9d, red dots) were examined, and the proportion of the major 5'-isomiR (i.e., isomiR with the same 5' end) was measured for quantification. The results indicated that changes in DICER's dsRBD primarily affected DICER cleavage sites, leading to alterations in the seed sequence of many miRNAs and/or strand switching (FIGS. 9d, 12a, and 12b). Similar changes were also observed in HEK293T cells (FIG. 10d). Validation through in vitro analysis using pre-miR-34a and pre-let-7e confirmed that their cleavage patterns significantly changed in a manner consistent with the sequencing data when dsRBD was deleted (FIGS. 9d and 13a to 13d). The changes in GYM motifs led to alterations in processing sites in these pre-miRNAs, supporting the importance of the GYM motif in cleavage site selection. Notably, these GYM variants could not be distinguished by ΔdsRBD mutants, indicating that the dsRBD-GYM interaction is crucial for determining cleavage sites in human pre-miRNA processing. Interestingly, GYM motif recognition appears to have differential effects on various miRNAs, either increasing (e.g., pre-miR-7-1 and pre-miR-30c) or decreasing (e.g., pre-miR-34a and pre-let-7e) cleavage homogeneity (FIGS. 12a, 12b, 13a, and 13b), likely due to the GYM motif cooperating with or competing against terminal recognition mechanisms depending on its relative position.

### EXAMPLE 5: Evolutionary Implications

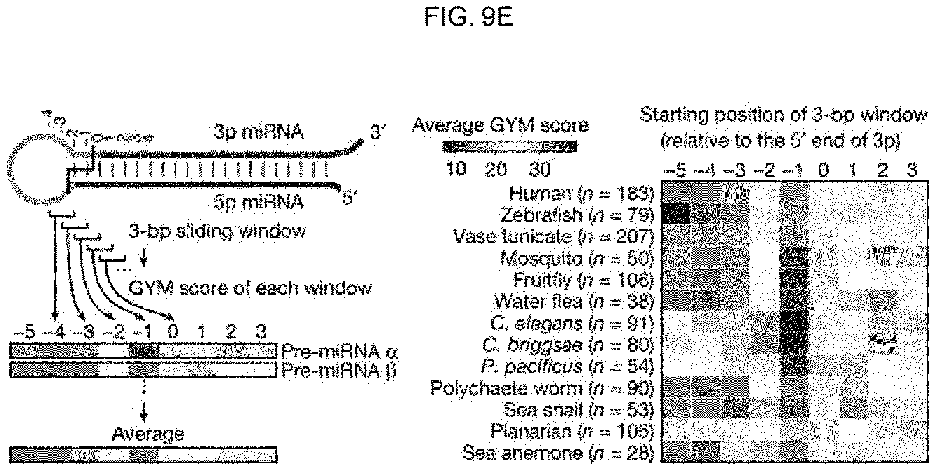

Given the overall and significant role of the GYM motif in miRNA maturation, the prominence of the GYM motif at the -1 to +1 positions across multiple pre-miRNAs and various species was analyzed as follows. The species analyzed included human *(Homo sapiens;* Hsa-miRNA), Zebrafish *(Danio rerio*; Dre-miRNA), vase tunicate *(Ciona intestinalis;* Cin-miRNA) mosquito *(Anopheles gambiae;* Aga-miRNA), fruitfly *(Drosophila melanogaster;* Dme-miRNA), water flea *(Daphina pulex;* Dpu-miRNA), *C. elegans (Caenorhabditis elegans;* Cel-miRNA), C. *briggsae* (*Caenorhabditis briggsae;* Cbr-miRNA), *P. pacificus* (*Pristionchus pacificus;* Ppc-miRNA), polychaete worm *(Capitella teleta;* Cte-miRNA), sea snail *(Lottia gigantea;* Lgi-miRNA), planarian *(Schmidtea mediterranea*; Sme-miRNA), and sea anemone *(Nematostella vectensis;* Nve-miRNA). As shown in FIG. 9e, it was found that in human pre-miRNAs, the GYM score at the -1 to +1 positions was higher than at adjacent positions (FIG. 9e, Human). This positional enrichment of GYM scores was observed in all metazoans analyzed (from sea anemones to humans). When combined with the biochemical data from Example 2, FIGS. 6c and 6d, showing that the activity of Drosophila Dicer-1 (Dcr-1) is promoted by the GYM motif, this pattern of positional conservation suggests that the GYM motif plays a deeply involved role in the evolution of miRNAs in metazoans.

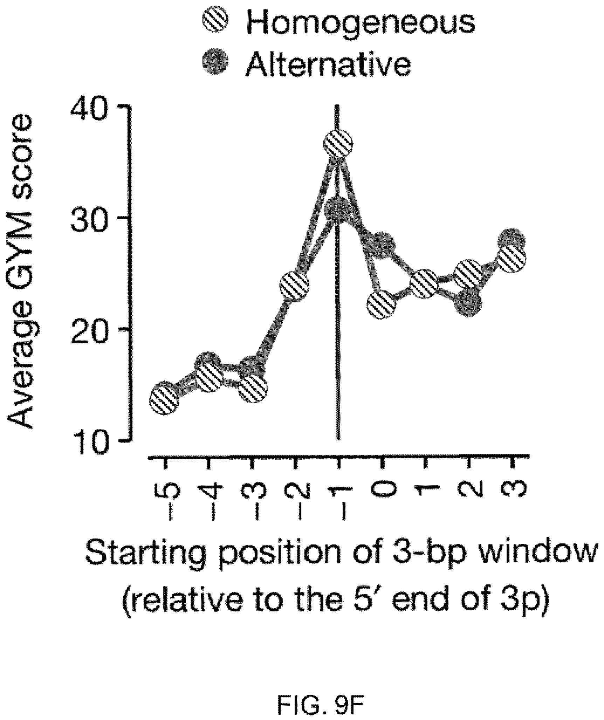

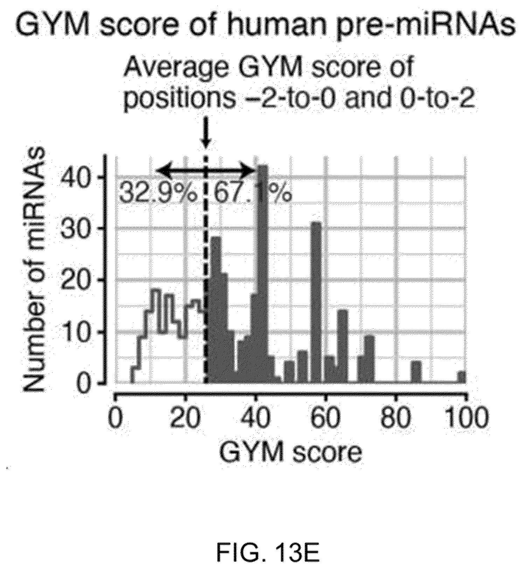

Specifically, as shown in FIG. 9f, miRNAs that are processed homogeneously had a more pronounced positional GYM score enrichment at a single site compared to alternatively processed miRNAs. Thus, a 'prominent' GYM motif relative to adjacent positions promotes the production of a single miRNA species, while a 'not-so-prominent' GYM motif allows for alternative processing, generating multiple isomiRs and diversifying miRNA functions. This suggests an explanation for why most human pre-miRNAs have evolved to have prominent GYM motifs, while some have evolved to have 'not-so-prominent' GYM motifs (FIG. 13e).

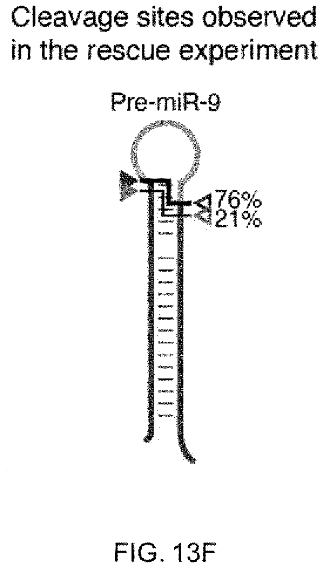

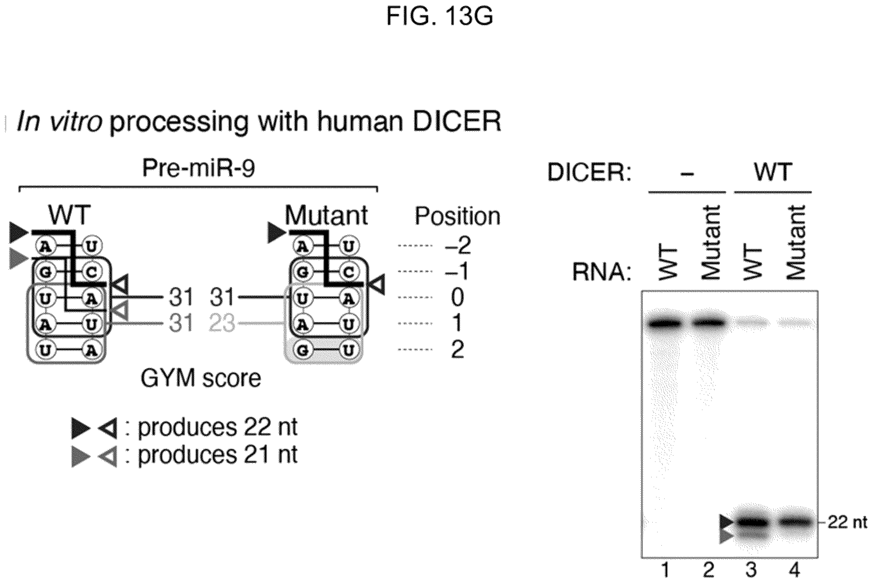

To experimentally test this hypothesis, pre-miR-9, which is selectively processed by DICER in cells to produce a major 22-nt isoform and a minor 21-nt isoform, was used as a model (FIG. 13f, blue and green arrows, respectively). As shown in FIG. 13f, pre-miR-9 lacks a prominent GYM motif, and the two 3-bp sliding windows corresponding to the two isomiRs have similar GYM scores (both around GYM score 31). By introducing a weaker GYM variant (with a GYM score of about 23) into the lower window without altering the upper window's GYM motif with a GYM score of about 31, the upper window was made more 'prominent' without raising its GYM score. As a result, pre-miR-9 was no longer subject to alternative processing (FIG. 13g). This indicates that the cause of alternative processing was the similarity in GYM scores between the two consecutive windows' GYM motifs. These experimental results demonstrate that the GYM motif can be used to promote either homogeneous processing or alternative processing of pre-miRNAs, depending on its relative strength and position.

### EXAMPLE 6: The GYM Motif Improves RNA Interference

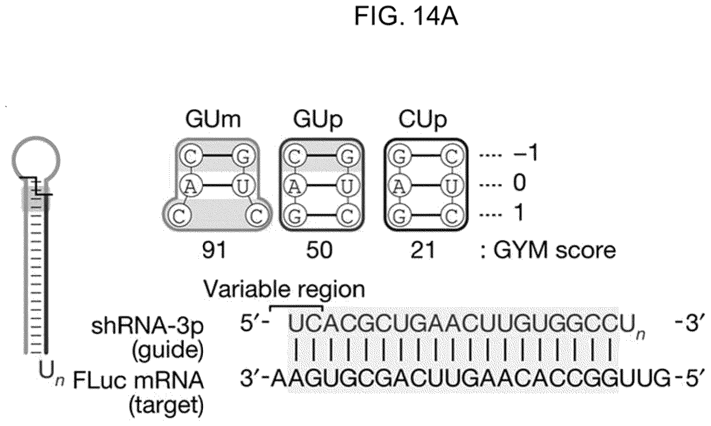

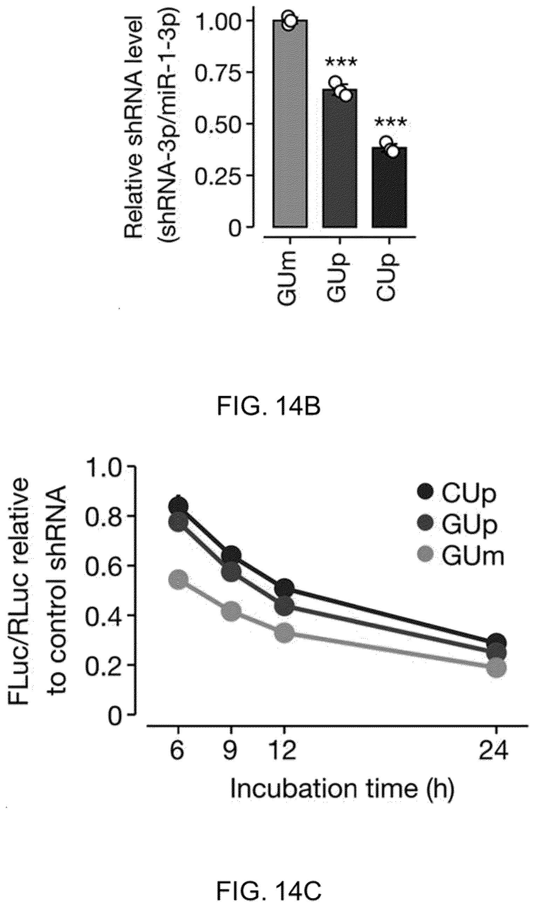

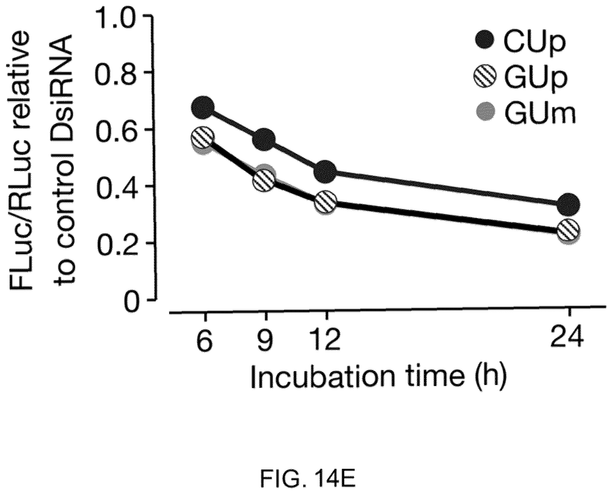

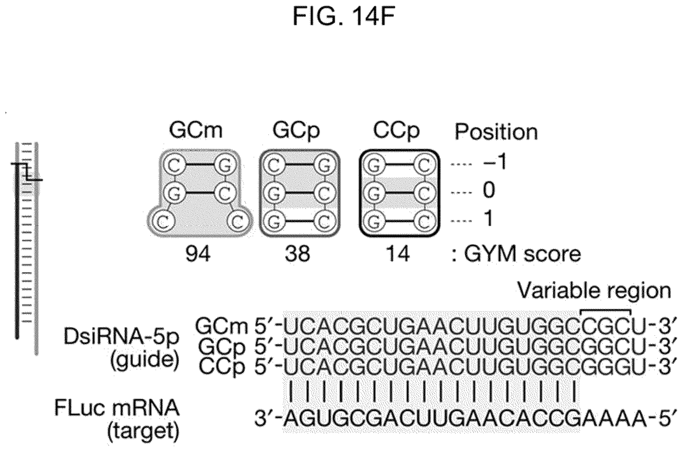

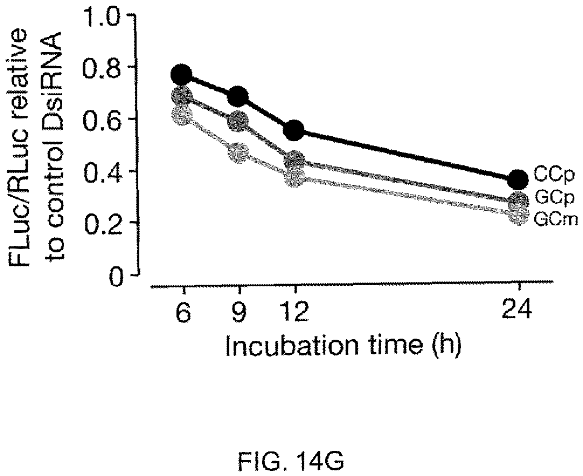

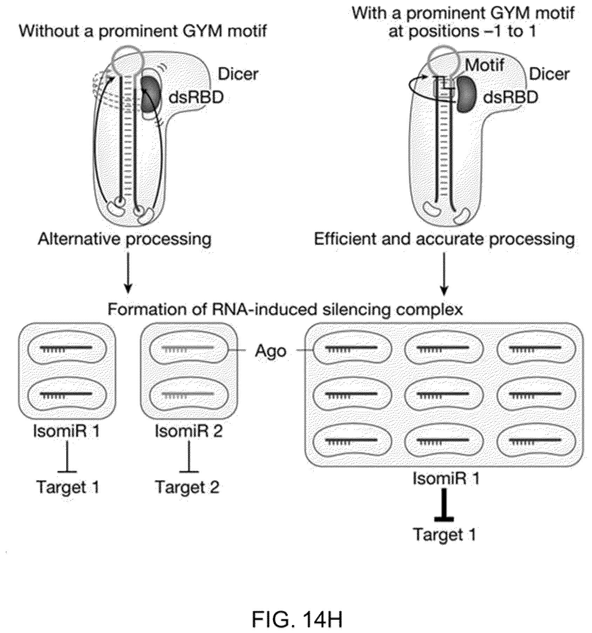

Based on the previous examples that established the GYM motif as a strong determinant of DICER-mediated dsRNA processing, the effect of the GYM motif on RNA-mediated interference using short hairpin RNA (shRNA) and DICER-substrate siRNA (DsiRNA) was investigated. Both shRNA and DsiRNA depend on cellular DICER to generate siRNA. shRNA is similar to pre-miRNA but includes a 5' triphosphate and a 3' overhang with three to five uridines derived from the termination signal of RNA polymerase III. This non-optimal terminal structure interferes with DICER processing, causing defects in shRNA-mediated interference. To test whether the GYM motif could override this terminal dependency, shRNAs with varying GYM motif scores were constructed (FIG. 14a). The shRNA with the highest GYM motif score produced the highest levels of siRNA (FIG. 14b) and exhibited the strongest gene silencing activity (FIG. 14c).

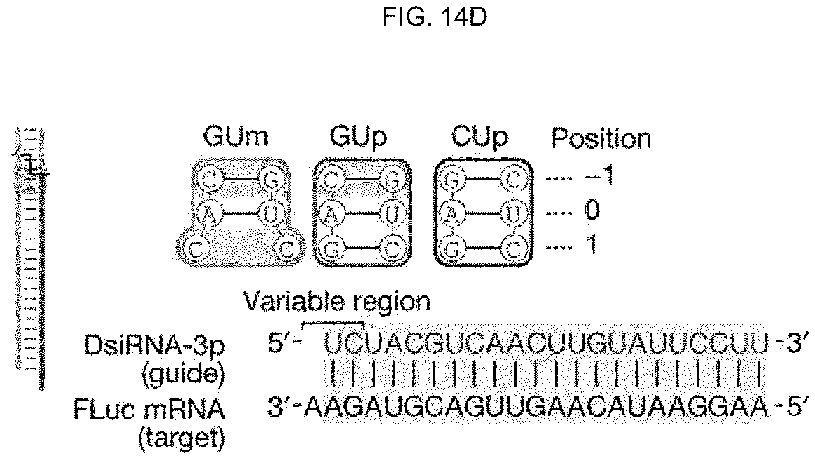

Additionally, the effect of the GYM motif was evaluated using DsiRNA-synthetic 27-nt siRNA duplexes designed to be processed by DICER into 22-nt siRNAs. DsiRNA exhibits stronger activity than synthetic 21-nt siRNA duplexes that bypass DICER processing, suggesting a link between DICER processing and Ago (Argonaute protein) loading. DsiRNAs can be designed to place the antisense guide strand on either the 5p or 3p strand. As shown in FIGS. 14d to 14g, a strong GYM motif enhanced knockdown efficiency regardless of whether the antisense strand was placed on the 5p or 3p strand. Collectively, these findings demonstrate that the GYM motif identified by the inventors enhances both shRNA-mediated and DsiRNA-mediated interference, enabling effective knockdown.

### EXAMPLE 7: Experimental Methods

### 1. Plasmid construction

To construct a plasmid for expressing human DICER, the coding sequence of human DICER (RefSeq NM_030621) was amplified and subcloned into a pX vector (a protein purification vector) containing an amino-terminal His10-eYFP-SUMOstar-Strep sequence or into a pCK vector (used for ectopic expression in DICER knockout cells) where the PGK promoter was replaced with a CMV promoter. Other DICER constructs, including DICER(ΔdsRBD) (amino acids 1 to 1,848), were generated through site-directed mutagenesis. For the plasmid expressing human TRBP, the coding sequence of human TRBP (RefSeq NM_134323) was amplified and subcloned into the same pX vector. For the plasmid expressing Drosophila Dcr-1, the coding sequence of Drosophila Dcr-1 (RefSeq NM_079729.3) was amplified from Drosophila cDNA and subcloned into the same pX vector. To construct a plasmid for dual-reporter luciferase assays, synthetic DNA oligonucleotides containing pri-miRNA sequences or target sites for shRNA and DsiRNA were inserted downstream of the FLuc gene in the pmirGLO vector (Promega). To construct plasmids for shRNA expression, synthetic DNA oligonucleotides containing shRNA sequences and pri-miR-1-1 sequences were inserted into within a vector, downstream of the U6 and UbC promoters, respectively.

### 2. Protein purification

N-terminal His10-eYFP-SUMOstar-Strep-tagged DICER expression plasmids were transiently transfected into HEK293E cells, derived from human embryonic kidneys, that were grown in suspension culture (Dulbecco's modified Eagle's medium (DMEM) supplemented with 5% fetal bovine serum) in an 8% CO₂ humidified shaking incubator at 37°C. At a cell density of about 7×10⁶ cells per mL, each plasmid was transfected at a final concentration of 0.3µg/ml with linear polyethylenimine and dimethylsulfoxide sequentially added at a final concentration of 3µg/ml and 1%, respectively. Transfected cells were incubated at 33°C for 72hours.

All purification steps were performed at 4°C. The cell pellet was resuspended in a buffer containing 100 mM Tris-HCl (pH 8.0), 150 mM NaCl, 10% glycerol, and 2 mM β-mercaptoethanol (BME), and supplemented with 0.1 mM phenylmethylsulfonyl fluoride (PMSF), EDTA-free Pierce Protease Inhibitor Mini Tablets (Thermo Fisher Scientific), 20 µl/ml micrococcal nuclease, and 5 mM CaCl2. The cells were lysed by sonication, and the lysate was centrifuged at 35,000 g for 1 hour. The supernatant was applied to Ni-NTA Superflow resin (Qiagen) pre-equilibrated with a buffer containing 100 mM Tris-HCl (pH 8.0), 150 mM NaCl, 10% glycerol, 2 mM BME, and 0.1 mM PMSF. The resin was washed with 5 column volumes of equilibration buffer supplemented with 40 mM imidazole. The bound protein was eluted with equilibration buffer supplemented with 200 mM imidazole. To cleave the N-terminal His10-eYFP-SUMOstar tag, the sample was treated with SUMOstar Protease (LifeSensors) for 1 hour at 4°C. The sample was then applied to Strep-Tactin Superflow resin (IBA Lifesciences) equilibrated with a buffer containing 100 mM Tris-HCl (pH 8.0), 150 mM NaCl, 2 mM BME, and 0.1 mM PMSF. The resin was washed sequentially with 5 column volumes of equilibration buffer containing 2 mM EDTA and 5 column volumes of equilibration buffer without EDTA. The bound protein was eluted with a buffer containing 100 mM Tris-HCl (pH 8.0), 150 mM NaCl, 2 mM BME, and 50 mM biotin. The eluate was applied to Ni-NTA Superflow resin (Qiagen) to remove uncleaved fusion protein. The unbound fractions were concentrated to approximately 20 µM using an Amicon Ultra-15 Centrifugal Filter Unit (100 kDa cutoff) and loaded onto a Superose 6 Increase 5/150 GL (GE Healthcare) column equilibrated with 50 mM Tris (pH 8.0), 100 mM NaCl, and 0.5 mM TCEP. The protein-containing fractions were pooled, concentrated to approximately 5 µM, snap-frozen in liquid nitrogen, and stored at -80°C until use. All human DICER constructs and Drosophila Dcr-1 were purified using the same method. Protein concentration was quantified by UV absorbance at 280 nm (NanoDrop) using the molecular extinction coefficient calculated for each protein.

### 3. Massively parallel assay to identify DICER processing determinants)

To predict the structure of dsRNA-bound human DICER, the cryo-EM structure of cleavage-incompetent human DICER was superimposed onto the crystal structure of dsRNA-bound A. aeolicus RNase III using PyMol. Based on the structural model, dsRNA regions that are likely recognized by dsRBD were predicted (FIG. 3a). Synthetic pre-miRNAs (Integrated DNA Technologies) with randomized nucleotides within the designated window, specifically pre-let-7a-1 and pre-miR-374b, were denatured at 80°C for 5 minutes and then slowly cooled to 4°C at a rate of -1°C/min in a buffer containing 20 mM Tris (pH 7.5), 80 mM NaCl, and 1 mM EDTA. The randomized pre-miRNA pool and purified DICER were mixed at final concentrations of 0.1 µM and 0.2 µM, respectively, in a reaction buffer containing 50 mM Tris (pH 7.5), 100 mM NaCl, 0.5 mM TCEP, and 2 mM MgCl2, to perform the massively parallel assay. The reaction was carried out at 37°C for a specified time to achieve approximately 10%, 20%, or 30% cleavage of the pre-miRNA pool. The reaction was terminated by adding an equal volume of 2X RNA loading dye (NEB) supplemented with 20 mM EDTA. Along with the input RNA, the mixture was resolved on a 15% urea-polyacrylamide gel. The input RNA and uncleaved pre-miRNA pool were gel-purified. The uncleaved pre-miRNA was then subjected to AQ-seq as previously described with slight modifications. After 3' adapter ligation, the ligated RNA was purified from a 10% urea-polyacrylamide gel along with a size marker, the Century-Plus RNA marker (Thermo Fisher). Following reverse transcription, cDNA was amplified using NEBNext Ultra II Q5 Master Mix (NEB). The libraries were sequenced on the NovaSeq 6000 platform.

The pre-miRNA to be sequenced were pre-processed by clipping the 3' adapter using cutadapt. Next, the 4-nt degenerate sequences were additionally removed using the FASTX-Toolkit (http://hannonlab.cshl.edu/fastx_toolkit/), and short, low-quality, and artefactual reads were filtered using the FASTX-Toolkit. Only pre-miRNAs without mutations outside the randomized region were selected for subsequent analysis. For a given pre-miRNA variant, the cleavage score was calculated by dividing the proportion in the input (with a pseudocount of 1) by the proportion in the uncleaved substrate sample.

### 4. DICER rescue experiment and data analysis

*DICER-knockout* HCT116 and HEK293T cells were maintained in DMEM and McCoy's 5A medium (WELGENE), respectively, supplemented with 10% fetal bovine serum (WELGENE). Both cell lines were authenticated using the ATCC short tandem repeat profiling and confirmed to be mycoplasma-negative. *DICER-knockout* HCT116 and HEK293T cells were transiently transfected with DICER expression plasmids using FuGENE HD (Promega) or Lipofectamine 3000 (Thermo Fisher), respectively. Total RNAs were extracted using TRIzol (Thermo Fisher) 48hours post-transfection and subjected to AQ-seq except that 100µg of total RNAs was used for library construction, and that cDNAs were amplified with NEBNext Ultra II Q5 Master Mix (NEB). The libraries were sequenced on the NovaSeq 6000 platform. Data processing was carried out as previously described. Briefly, the 3' adapter and 4-nt degenerate sequences were removed from the pre-miRNA using cutadapt46 and FASTX-Toolkit (http://hannonlab.cshl.edu/ fastx_toolkit/), respectively. Next, short, low-quality and artefactual reads were filtered out using FASTX-Toolkit. The output reads were first mapped to the spike-in sequences, and then unmapped reads were aligned to the human genome (hg38) using BWA50. Corresponding miRNA annotations were found with miRBase release 21 using the intersect tool in BEDTools. For the analysis of miRNA abundance, read counts of miRNAs were normalized with the average of read counts of spike-ins. For the analysis of processing accuracy, the most abundant 5'-isomiR was identified for a given mature miRNA in samples expressing WT DICER. Then the proportions of the 5'-isomiR were measured for all of the samples. Fold changes of the proportions between samples were calculated to examine processing accuracy defects. To reduce the influence of decay intermediates, exclusion from subsequent analysis was made of miRNAs that do not have any 5'-isomiR accounting for more than 20% of the 5'-isomiRs with 5' ends within positions -5 to 5, relative to the annotated one.

### 5. Analysis on GYM motif of natural miRNAs

Representative animal miRNAs from various species were used for the pri-miRNA motif conservation analysis. A curated list (see Fang, Wenwen, and David P Bartel. "The Menu of Features that Define Primary MicroRNAs and Enable De Novo Design of MicroRNA Genes." Molecular Cell, vol. 60, no. 1, 2015, pp. 131-45, and Fromm, Bastian, et al. "MirGeneDB 2.0: The Metazoan MicroRNA Complement." Nucleic Acids Research, vol. 48, no. D1, 2020, pp. D132-D141) was adopted and further curated as follows: miRNA annotations and sequences were manually curated based on miRBase release 22 (refer to Kozomara, Ana, and Sam Griffiths-Jones. "miRBase: Annotating High Confidence MicroRNAs Using Deep Sequencing Data." Nucleic Acids Research, vol. 42, no. D1, 2014, pp. D68-D73); pre-miRNA sequences were retrieved and added to the list based on 5p and 3p annotations from miRBase release 22; pre-miRNA sequences from miRGeneDB release 2.0 were added to the list; 3p sequences from miRBase release 22 and miRGeneDB release 2.0 were added to the list; for each miRNA, if a 3p sequence was available, the start position of the 3p miRNA was identified, and if not, the 3p sequence and start position were manually curated based on homologs from closely related species. The secondary structure of pre-miRNA was obtained using the default settings of Fold in RNAstructure version 6.3. Based on the predicted structure for a given miRNA, the 3-bp configuration at each position was identified relative to the start position of the 3p strand (see FIG. 9e). Next, the average GYM score for the 3 bp at the -1 to +1 positions was calculated using the scores obtained from the massively parallel assay of two pre-miRNA backbones that were randomized at these positions under conditions where approximately 20% of the substrate was cleaved. Pre-miRNA sequences were prioritized and referenced from miRGeneDB, miRBase, and the sequences listed in Fang et al., 2015. The 3p positions were prioritized and referenced from miRGeneDB, miRBase, and the positions manually curated by the inventors. If a bulge or mismatch was present at a specific position, that position was excluded. If no usable 3p position was available, the miRNA was excluded from further analysis.

### 6. Dual luciferase reporter assay for gene silencing activity

For shRNA, HEK293T cells were co-transfected using Lipofectamine 3000 reagent (Thermo Fisher) with plasmids encoding shRNA and pri-miR-1-1 under the control of independent promoters, along with the pmirGLO vector containing the target site of the shRNA-3p. The shRNA was optimally designed according to established guidelines. For DsiRNA, HEK293T cells were co-transfected using Lipofectamine 2000 reagent (Thermo Fisher) with DsiRNA (Integrated DNA Technologies) and the pmirGLO vector containing the target site of the DsiRNA. Cells were collected at different time points post-transfection and the luciferase signals were measured by the Dual Luciferase Reporter Assay System according to the manufacturer's instructions (Promega) on a Spark Multimode Microplate Reader (TECAN).

### 7. Quantitative real-time PCR

HEK293T cells were transfected as described above. RNAs were isolated at various time points post-transfection using TRIzol (Thermo Fisher) and the Direct-zol RNA Miniprep Kit (Zymo Research), following the manufacturer's instructions. cDNA was synthesized using the TaqMan miRNA Reverse Transcription Kit (Applied Biosystems) and quantitative real-time PCR was performed using the TaqMan MicroRNA Assay (Applied Biosystems) on the StepOnePlus Real-Time PCR System (Thermo Fisher). Given that miR-1-3p is expressed at low levels in HEK293T (about 8 RPM in the AQ-seq result)25, miR-1-3p was used as an internal control.

### 8. Statistics and reproducibility

All in vitro assays were repeated more than twice (FIGS. 6a to 6e, 6g, 6h, 9c, 1c, 3c, 7a to 7c, 7e to 7g, 8a, 8c to 8f, 11a to 11c, 13b, 13d, and 13g).

## Claims

1. A DICER cleavage site motif, comprising a 5' arm with a 5'-N₁N₂N₃-3' nucleic acid sequence and a 3' arm with a 5'-N_{3'}N_{2'}N_{1'}-3' nucleic acid sequence,
wherein cleavage by DICER occurs between N3' and N2' of the 3' arm; and
a pair of 5'-N₁N₂N₃-3' nucleic acid sequence and 5'-N_{3'}N_{2'}N_{1'}-3' nucleic acid sequence that constitute the DICER cleavage site motif is any one selected from the group consisting of the following nucleic acid sequence pairs:
5'-CGC-3'/5'-GCG-3';
5'-AGC-3'/5'-GCA-3';
5'-CGC-3'/5'-GCC-3';
5'-CAC-3'/5'-GUC-3';
5'-AAU-3'/5'-AUU-3';
5'-CGC-3'/5'-GUC-3';
5'-AGC-3'/5'-GCU-3';
5'-AGU-3'/5'-ACA-3';
5'-AAC-3'/5'-GUA-3';
5'-AGC-3'/5'-GCG-3';
5'-GGC-3'/5'-GCG-3';
5'-AAC-3'/5'-GUC-3';
5'-UAC-3'/5'-GCA-3';
5'-UUC-3'/5'-GUA-3';
5'-AUU-3'/5'-AAA-3';
5'-UGC-3'/5'-GCC-3';
5'-AGC-3'/5'-GCC-3';
5'-CGG-3'/5'-CUC-3';
5'-UGC-3'/5'-GCA-3';
5'-CAC-3'/5'-GUG-3';
5'-CGU-3'/5'-ACC-3';
5'-AAC-3'/5'-GUU-3';
5'-GGC-3'/5'-GCA-3';
5'-AAC-3'/5'-GCU-3';
5'-CAU-3'/5'-AUC-3' ;
5'-AUC-3'/5'-GAU-3' ;
5'-UGC-3'/5'-GCU-3';
5'-UGC-3'/5'-GUC-3';
5'-UUC-3'/5'-GUG-3';
5'-CGC-3'/5'-GCU-3';
5'-AGC-3'/5'-GUC-3';
5'-ACC-3'/5'-GGA-3';
5'-UUC-3'/5'-GAA-3' ;
5'-CUC-3'/5'-GAC-3';
5'-CUU-3'/5'-AAC-3' ;
5'-AAC-3'/5'-GUG-3' ;
5'-CCC-3'/5'-GGC-3';
5'-AGU-3'/5'-ACC-3';
5'-CAC-3'/5'-GCG-3';
5'-AGU-3'/5'-ACG-3'; and
5'-UAC-3'/5'-GUA-3'.

2. The DICER cleavage site motif of claim 1, wherein the DICER cleavage site motif is included in a double-stranded RNA (dsRNA) and promotes the processing of the dsRNA by DICER.

3. The DICER cleavage site motif of claim 2, wherein the dsRNA is any one selected from the group consisting of pri-miRNA (primary miRNA), pre-miRNA (precursor miRNA), shRNA (short hairpin RNA), DsiRNA (Dicer-substrate short interfering RNA), and long dsRNA.

4. The DICER cleavage site motif of claim 1, wherein the DICER cleavage site motif promotes biogenesis of miRNA or siRNA.

5. The DICER cleavage site motif of claim 1, wherein the DICER cleavage site motif promotes RNA interference.

6. A dsRNA nucleic acid molecule, comprising the DICER cleavage site motif according to any one of claims 1 to 5.

7. The dsRNA nucleic acid molecule of claim 6, wherein the dsRNA nucleic acid molecule:
comprises a stem of 22 base pairs (bp) or longer:
comprises the DICER cleavage site motif at a position between 18^{th} to 20^{th}, 19^{th} to 21^{st}, or 20^{th} to 22^{nd} position from the 5' end;
forms a double strand with or without one or more mismatches in the stem; and
generates a miRNA or siRNA of 21 to 23 nucleotides in length through cleavage by DICER.

8. The dsRNA nucleic acid molecule of claim 7, wherein the dsRNA nucleic acid molecule is any one selected from the group consisting of pri-miRNA (primary miRNAs), pre-miRNA (precursor miRNAs), shRNA (short hairpin RNA), DsiRNA (Dicer-substrate short interfering RNA), and long dsRNA.

9. A method for enhancing capacity of RNA interference of a target double-stranded RNA (dsRNA), the method comprising the steps of:
(a) calculating a GYM score of a DICER cleavage site motif within the target dsRNA; and
(b) replacing the DICER cleavage site motif within the target dsRNA with a second DICER cleavage site motif that has a relatively higher GYM score than the GYM score of the DICER cleavage site motif within the target dsRNA, calculated in step (a).

10. A method for promoting homogeneous processing of double-stranded RNA (dsRNA), the method comprising the steps of:
(a) forming a DICER cleavage site motif in a target dsRNA, wherein the cleavage by DICER generates a desired nucleic acid molecule;
wherein a GYM score of the DICER cleavage site motif is relatively higher compared to the GYM scores of all nucleic acid sequences determined by a 1 nt to 2 nt frame shift in both directions based on the region of the DICER cleavage site motif.

11. A method for promoting alternative processing of double-stranded RNA (dsRNA), the method comprising the steps of:
(a) replacing a nucleic acid of a DICER cleavage site motif region in a target dsRNA so that a difference in GYM score between a major DICER cleavage site motif, which has a highest GYM score among multiple adjacent DICER cleavage site motifs, and at least one adjacent DICER cleavage site motif is less than 8.
